# EUROPÄISCHE PATENTSCHRIFT

(11) **EP 0 850 216 B1**
(45) Veröffentlichungstag und Bekanntmachung des Hinweises auf die Patenterteilung: **28.03.2001**
(21) Anmeldenummer: 96926379.7
(22) Anmeldetag: 22.07.1996
(51) Int. Cl.: C07C 259/06, C07D 261/08, C07C 327/58, A01N 37/36, A01N 37/50

(54) **HYDROXIMSÄUREDERIVATE, VERFAHREN ZU IHRER HERSTELLUNG UND IHRE VERWENDUNG ALS SCHÄDLINGSBEKÄMPFUNGSMITTEL UND FUNGIZIDE**
HYDROXIMIC ACID DERIVATIVES, A PROCESS FOR PREPARING THE SAME AND THEIR USE AS PESTICIDES AND FUNGICIDES
DERIVES D'ACIDE HYDROXIMIQUE, LEUR PROCEDE DE PREPARATION ET LEUR UTILISATION COMME PESTICIDES ET FONGICIDES

(30) Priorität: 04.08.1995 DE 19528651
(43) Veröffentlichungstag der Anmeldung: 01.07.1998
(73) Patentinhaber: BASF AKTIENGESELLSCHAFT, 67056 Ludwigshafen (DE)
(72) Erfinder: BAYER, Herbert, D-68159 Mannheim (DE); MÜLLER, Ruth, D-67159 Friedelsheim (DE); SAUTER, Hubert, D-68167 Mannheim (DE); HARREUS, Albrecht, D-67063 Ludwigshafen (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE)
(86) Internationale Anmeldenummer: EP9603218
(87) Internationale Veröffentlichungsnummer: WO9706133

(56) Entgegenhaltungen:
- EP-A- 0 848 701
- WO-A-95/18789
- WO-A-95/21153
- WO-A-95/21154
- WO-A-97/01530

## Beschreibung

Die vorliegende Erfindung betrifft Hydroximsäurederivate der Formel I 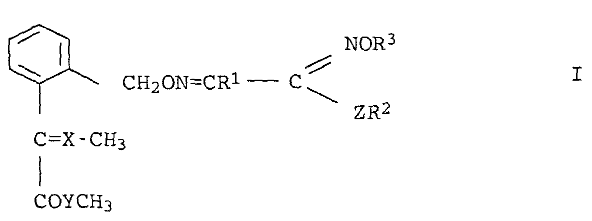 in der die Substituenten die folgende Bedeutung haben:
- X: CH, CHO oder NO;
- Y: O oder NH;
- Z: O oder S;
- R¹: Wasserstoff, C₁-C₆-Alkyl oder C₁-C₄-Halogenalkyl;
- R²: C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
- Cyano, Nitro, Amino, Hydroxy,
- C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl, C₁-C₄-Alkyl, C₁-C₄-Halogenalkyl, C₁-C₄-Alkylcarbonyl, C₁-C₆-Alkoxy, C₁-C₆-Halogenalkoxy, C₁-C₆-Alkoxycarbonyl, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino, C₁-C₆-Alkylaminocarbonyl, Di-C₁-C₆-alkylaminocarbonyl, C₂-C₆-Alkenyloxy und Phenyl;
   C₃-C₆-Cycloalkyl, wobei diese Gruppen partiell oder vollständig halogeniert sein können und/oder einen bis drei der folgenden Reste tragen können:
- Cyano, Nitro, Amino, Hydroxy,
- C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino;
- R³: C₁-C₆-Alkyl, C₂-C₆-Alkenyl oder C₂-C₆-Alkinyl, wobei diese Gruppen partiell oder vollständig halogeniert sein können und/oder einen bis drei der folgenden Reste tragen können:
- Cyano, C₁-C₃-Alkoxy,
- Oxiranyl oder C₃-C₆-Cycloalkyl, wobei die cyclischen Reste ihrerseits partiell oder vollständig halogeniert sein können und/oder eine bis drei C₁-C₄-Alkylgruppen tragen können;
ausgenommen Hydroximsäurederivate der Formel I, in der
a)
   X NO und
   Y O oder NH bedeuten; oder
b)
   X CHO und
   Y O bedeuten;

R¹, R³ Methyl; und
R² Ethyl, 2,2,2-Trifluorethyl, 2-Chlorethyl, n-Propyl, i-Pentyl, tert.-Pentyl oder n-Hexyl bedeuten.

Außerdem betrifft die Erfindung Verfahren zr Herstellung dieser Verbindungen sowie sie enthaltende Mittel zur Bekämpfung von tierischen Schädlingen und Schadpilzen.

Aus der Literatur sind Phenylessigsäurederivate zur Bekämpfung von tierischen Schädlingen und Schadpilzen bekannt (EP-A 422 597, EP-A 463 488, EP-A 370 629, EP-A 460 575, EP-A 472 300, WO-A 90/07,493, WO-A 92/13,830, WO-A 92/18,487). In der WO-A 95/18789 sind Phenylessigsäurederivate beschrieben, die in ortho-Stellung zur Essigsäurefunktion am Phenylring eine Bisoxim-Seitenkette tragen. Die erfindungsgemäßen Verbindungen hingegen weisen am Ende der Seitenkette eine Hydroximsäurefunktion auf.

Einzelne Phenylessigsäurederivate, die in der Seitenkette eine Hydroximsäurefujnktion aufweisen, sind in der nachveröffentlichten WO-A 97/01530 beschrieben. Außerdem werden in den älteren Patentanmeldungen WO-A 95/21153 und WO-A 95/21154 Phenylessigsäurederivate mit Bis-Oxim Seitenketten mit Wirkung gegen tierische Schädlinge und Schadpilze beschrieben. Demgegenüber lagen der vorliegenden Erfindung Verbindungen mit verbesserter Wirkung als Aufgabe zugrunde.

Demgemäß wurden sie eingangs definierten Hydroximinsäurederivate I gefunden. Außerdem wurden Verfahren zu ihrer Herstellung sowie sie enthaltende Mittel zur Bekämpfung von tierischen Schädlingen und Schadpilzen gefunden.

Die Verbindungen I sind auf verschiedenen Wegen nach an sich in der Literatur bekannten Verfahren erhältlich. Grundsätzlich ist es bei der Synthese der Verbindungen I unerheblich, ob zunächst die Gruppierung -C(=XCH₃)-COYCH₃ oder die Gruppe -CH₂ON=CR¹-C(ZR²)=NOR³ aufgebaut wird. Die verschiedenen Methoden zum Aufbau der -C(=XCH₃)-COYCH₃-Gruppierung (in den nachfolgenden Formeln z.T. mit dem Zeichen # verkürzt dargestellt) sind beispielsweise aus der eingangs zitierten Literatur bekannt.

Beim Aufbau der Gruppe -CH₂ON=CR¹-C(ZR²)=NOR³ geht man im allgemeinen so vor, daß man ein Benzylderivat der Formel II mit einem Hydroxyimin der Formel III umsetzt.

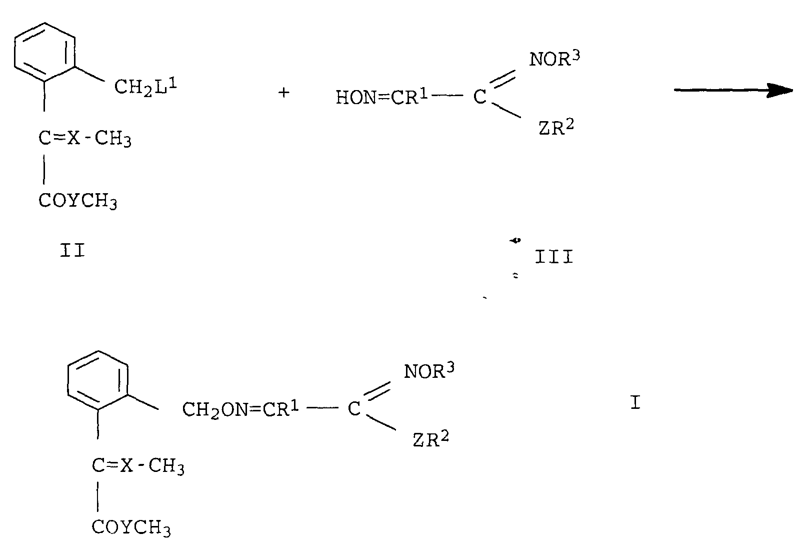

L¹ in der Formel II steht für eine nucleophil austauschbare Abgangsgruppe, z.B. Halogen oder Sulfonatgruppen, vorzugsweise Chlor, Brom, Iod, Mesylat, Tosylat und Triflat.

Die Umsetzung erfolgt in an sich bekannter Weise in einem inerten organischen Lösungsmittel in Gegenwart einer Base (z.B. Natriumhydrid, Kaliumhydroxid, Kaliumcarbonat und Triethylamin) gemäß den in Houben-Weyl, Bd. E 14b, S. 370f und Houben-Weyl, Bd. 10/1, S. 1189f beschriebenen Methoden.

Das benötigte Hydroxyimin III erhält man beispielsweise durch Umsetzung eines entsprechenden Dihydroxyimins IV mit einem nucleophil substituierten Reagens VIa.

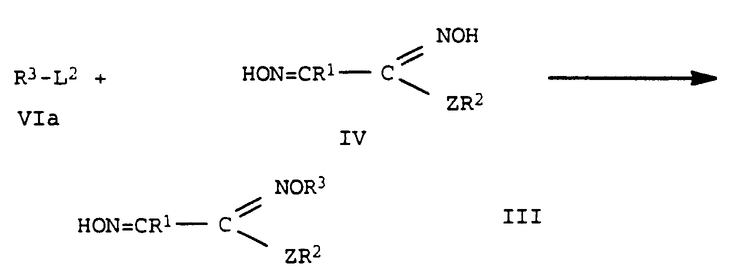

L² in der Formel VIa steht für eine nucleophil austauschbare Abgangsgruppe, z.B. Halogen oder Sulfonatgruppen, vorzugsweise Chlor, Brom, Iod, Mesylat, Tosylat und Triflat.

Die Umsetzung erfolgt in an sich bekannter Weise in einem inerten organischen Lösungsmittel in Gegenwart einer Base (z.B. Kaliumcarbonat, Kaliumhydroxid, Natriumhydrid, Pyridin und Triethylamin) gemäß den in Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f beschriebenen Methoden.

Die Verbindungen IV sind bekannt (DE-A 26 21 102) oder können nach bekannter Methoden hergestellt werden.

Alternativ können die Verbindungen I auch dadurch erhalten werden, daß man das Benzylderivat II zunächst mit einem Dihydroxyimin IV in ein entsprechendes Benzyloxim V überführt und V anschließend mit dem nucleophil substituierten Reagens VIa zu I umsetzt.

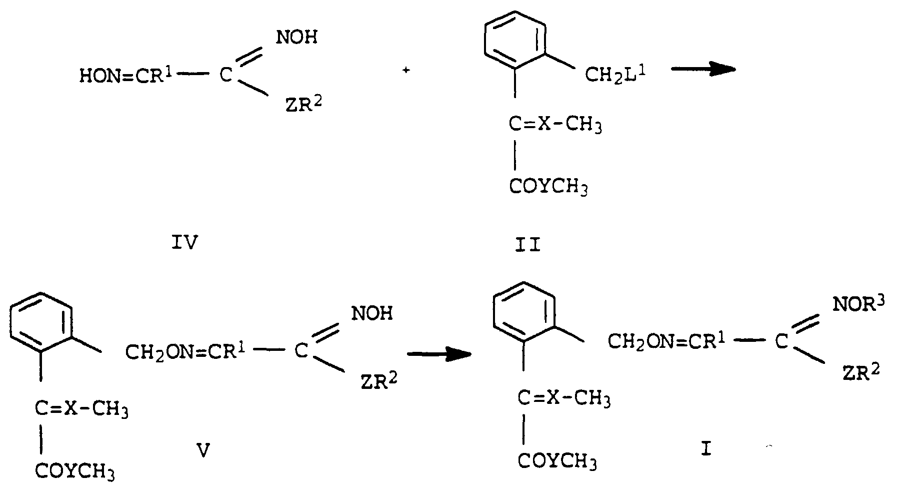

Die Umsetzung erfolgt in an sich bekannter Weise in einem inerten organischen Lösungsmittel in Gegenwart einer Base (z.B. Kaliumcarbonat, Kaliumhydroxid, Natriumhydrid, Pyridin und Triethylamin) gemäß den in Houben-Weyl, Bd. 10/1, S. 1189f; Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f beschriebenen Methoden.

Nach einem weiteren Verfahren können die Verbindungen I auch dadurch erhalten werden, daß man das Benzylderivat II zunächst mit einem Hydroxyiminoester der Formel VII in den Benzyloxyiminoester VIIIa überführt und VIIIa zur entsprechenden Säure VIIIb verseift. Durch Umsetzung der Säure VIIIb mit einem Hydroxylamin der Formel IXa oder dessen Salz IXb erhält man die entsprechende Hydroxamsäure Xa, die mit üblichen Halogenierungsmitteln in die entsprechenden Hydroxamsäurehalogenide Xb überführt werden können. Aus den Hydroxamsäurehalogeniden Xb erhält man durch Umsetzung mit nucleophilen Verbindungen XI die Verbindungen I.

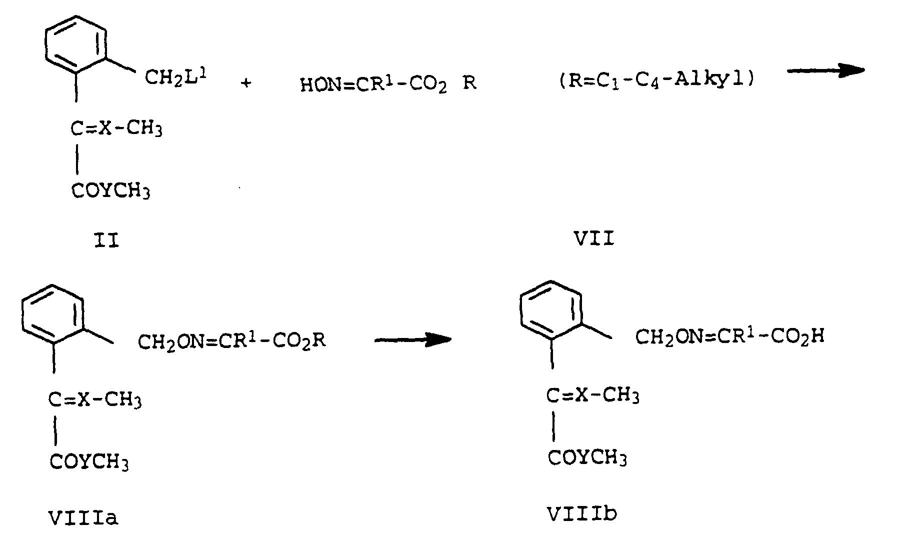

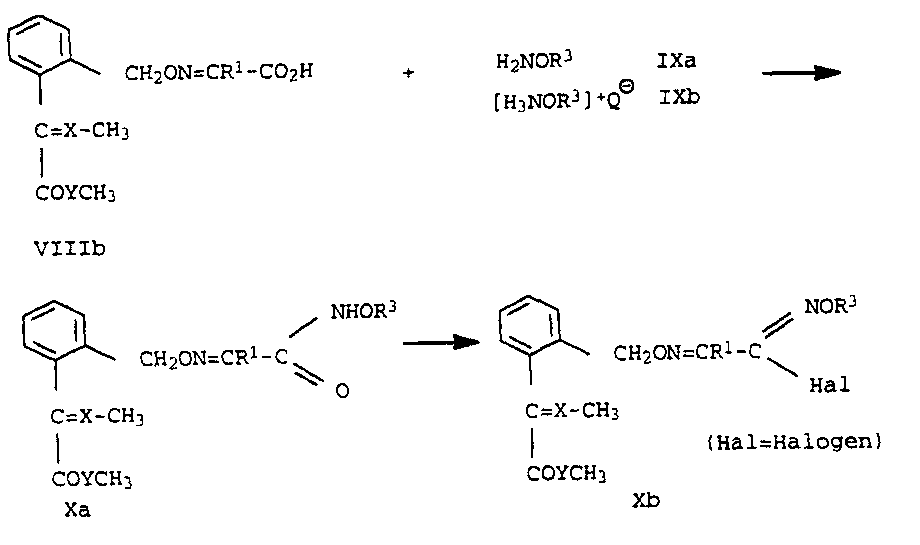

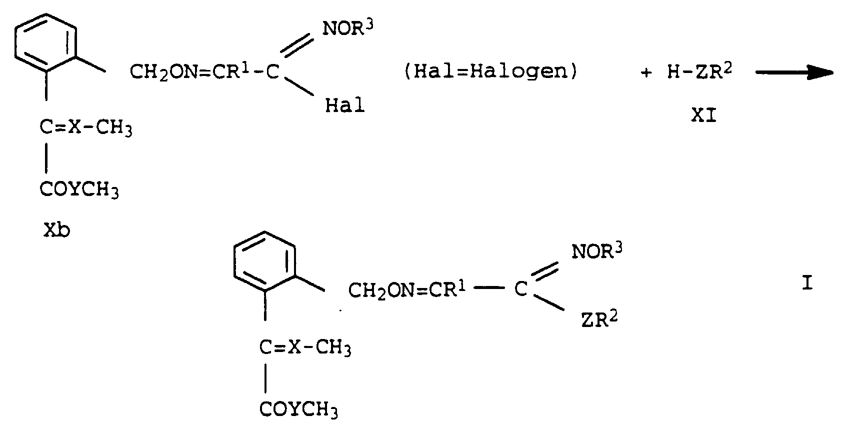

R in den Formeln VII und VIIIa steht für eine C₁-C₄-Alkylgruppe (insbesondere Methyl, Ethyl, Isopropyl und tert.-Butyl); Q⁻ in der Formel IXb bedeutet das Anion einer anorganischen Säure (insbesondere ein Halogenid wie Chlorid); Hal in der Formel Xb steht für ein Halogenatom wie Chlor, Brom und Iod.

Die Umsetzung des Benzylderivats II mit dem Hydroxyiminoester VII erfolgt im wie im allgemeinen und im besonderen für die Umsetzung von II mit III beschrieben.

Die Verbindungen VII sind bekannt [J. Am. Chem. Soc. 100, 1857 (1978); Bull. Soc. Chim. Fr. 1975, 252; Bull. Soc. Chim. Fr. 31, 1054 (1904); US-A 3,584,032] oder können nach den dort beschriebenen Methoden erhalten werden.

Die Verseifung des Esters VIIIa zu VIIIb erfolgt analog literaturbekannten Methoden (vgl. Houben-Weyl Bd. E 5, S. 223f) bei Temperaturen von 0°C bis 150°C, vorzugsweise 20°C bis 80°C in Gegenwart einer Säure oder Base.

Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Wasser, Methanol und Ethanol.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetallcarbonate wie Lithiumcarbonat und Calziumcarbonat sowie Alkalimetallhydrogencarbonate wie Natriumhydrogencarbonat, in Betracht.

Besonders bevorzugt werden NaOH und KOH.

Die Basen werden im allgemeinen äquimolar oder im Überschuß eingesetzt.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propion-säure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt.

Die Umsetzung der Säure VIIIb mit einem Hydroxylamin der Formel IXa oder dessen Salz IXb erfolgt üblicherweise bei Temperaturen von -10°C bis 120°C, vorzugsweise 0°C bis 50°C, in Gegenwart von Aktivierungsreagentien gemäß den in Houben-Weyl Bd. E 5, S. 1141f beschriebenen Methoden.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran.

Es können auch Gemische der genannten Lösungsmittel verwendet werden.

Als Aktivierungsreagentien eignen sich Säurehalogenidbildner wie Phosgen, Phosphortribromid, Phosphortrichlorid, Phosphorpentachlorid, Phosphoroxychlorid, Thionylchlorid oder Oxalylchlorid; Anhydridbildner wie Chlorameisensäureethylester oder Methansulfonylchlorid; Carbodiimide wie N,N'-Dicyclohexylcarbodiimid oder andere übliche Mittel wie N,N'-Carbonyldiimidazol oder Triphenylphosphin in Tetrachlorkohlenstoff.

Besonders bevorzugt werden Thionylchlorid, Oxalylchlorid, N,N'-Carbonyldiimidazol.

Die Aktivierungsreagentien werden im allgemeinen äquimolar oder im Überschuß verwendet.

Bei der anschließenden Halogenierung der Hydroxamsäure Xa zu den Halogeniden Xb verwendet man übliche Halogenierungsmittel (z.B.Thionylchlorid, Oxalylchlorid, Phosphortribromid, Phosphortrichlorid, Phosphorpentachlorid, Phosphoroxychlorid, Triphenylphosphin und Tetrachlorkohlenstoff bzw. Tetrabromkohlenstoff); die Umsetzung erfolgt üblicherweise bei Temperaturen von -20°C bis 100°C, vorzugsweise -10°C bis 80°C gemäß bekannten Verfahren [Houben-Weyl Bd. E5, S. 631; J. Org. Chem. 36, 233 (1971); J. Org. Chem. 57, 3245 (1992)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid und Dimethylformamid, besonders bevorzugt Acetonitril, Toluol und Tetrahydrofuran.

Es können auch Gemische der genannten Lösungsmittel verwendet werden.

Die Edukte werden im allgemeinen in äquimolaren Mengen miteinander umgesetzt. Es kann für die Ausbeute vorteilhaft sein, das Halogenierungsmittel in einem Über- oder Unterschuß bezogen auf Xa einzusetzen.

Aus den so erhaltenen Hydroxamsäurehalogeniden Xb erhält man die Verbindungen I durch Umsetzung mit nucleophilen Verbindungen XI gemäß allgemein bekannten Verfahren [Houben-Weyl Bd. E 5, S. 826f und S. 1280f; J. Org. Chem. 36, 233 (1971); Collect. Czech. Chem. Commun. 48, 596 (1983); J. Org. Chem. 46, 3623 (1981)], üblicherweise bei Temperaturen von 0°C bis 150°C, vorzugsweise 20°C bis 100°C in Gegenwart einer Base.

Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid und Dimethylformamid, besonders bevorzugt Dimethylformamid, Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydrid, Kaliumhydrid, Kaliumcarbonat, Triethylamin und Pyridin.

Die Basen werden im allgemeinen äquimolar, im Überschuß oder gegebenenfalls als Lösungsmittel verwendet.

Die Edukte werden im allgemeinen in äquimolaren Mengen miteinander umgesetzt. Es kann für die Ausbeute vorteilhaft sein, XI in einem Überschuß bezogen auf Xb einzusetzen.

Verbindungen I, in denen Z für Sauerstoff steht, erhält man besonders bevorzugt im Sinne des vorstehend beschriebenen Verfahrens dadurch, daß man die Hydroxamsäuren Xa direkt in Gegenwart einer Base in einem inerten organischen Lösungsmittel mit einer Verbindung VIb umsetzt [vgl. Houben-Weyl Bd. E 5, S. 826f; Aust. J. Chem. 27, 1341 (1974)].

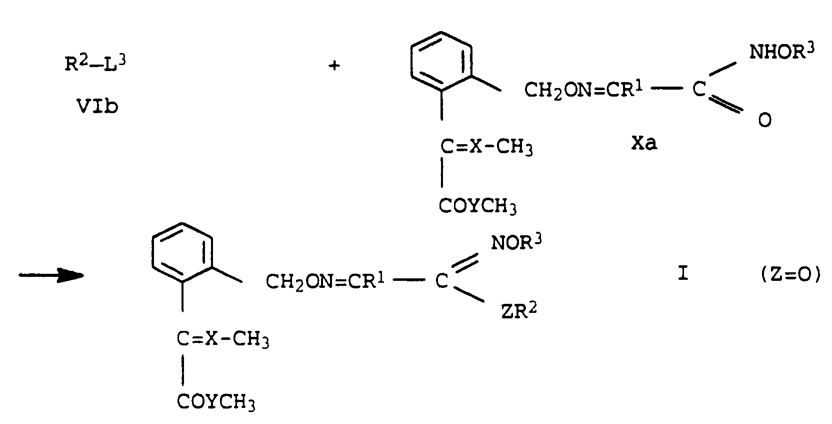

L³ in der Formel VIb steht für eine nucleophil austauschbare Abgangsgruppe, z.B. Halogen oder Sulfonatgruppen, vorzugsweise Chlor, Brom, Iod, Mesylat, Tosylat und Triflat.

Diese Umsetzung erfolgt üblicherweise bei Temperaturen von 0°C bis 150°C, vorzugsweise 20°C bis 180°C.

Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid und Dimethylformamid, besonders bevorzugt Dimethylformamid und Tertrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Kaliumcarbonat, Kaliumhydroxyd, Natriumhydrid, Pyridin und Triethylamin.

Die Basen werden im allgemeinen in äquimolar, im Überschuß oder gegebenenfalls als Lösungsmittel verwendet.

Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung XII.

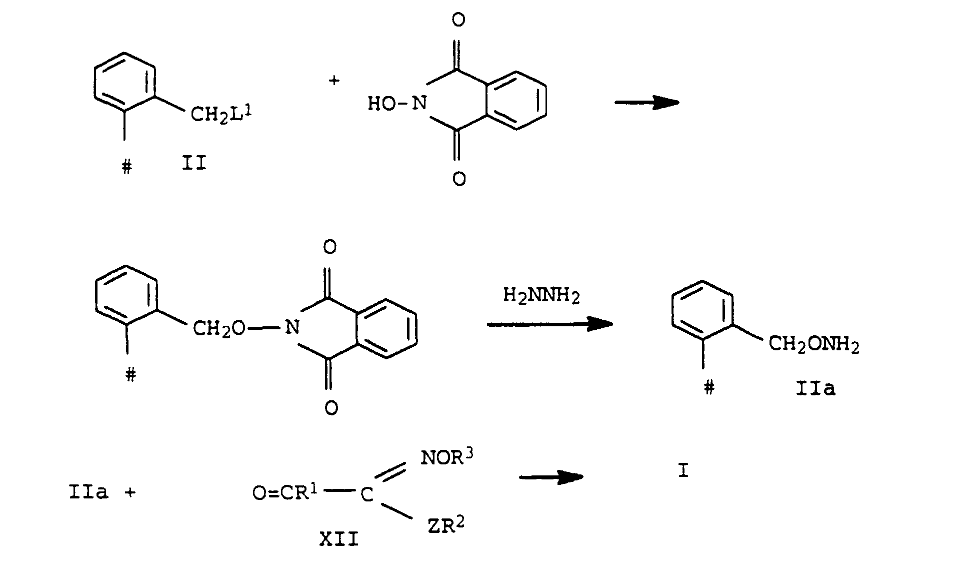

Die Umsetzung erfolgt in an sich bekannter Weise in einem inerten organischen Lösungsmittel gemäß den in EP-A 463 488 und EP-A 585 751 beschriebenen Methoden.

Die benötigten Carbonylverbindungen XII erhält man beispielsweise durch Umsetzung der entsprechenden Hydroxyiminocarbonylverbindungen XIIa mit einem nucleophil substituierten Reagens VIa.

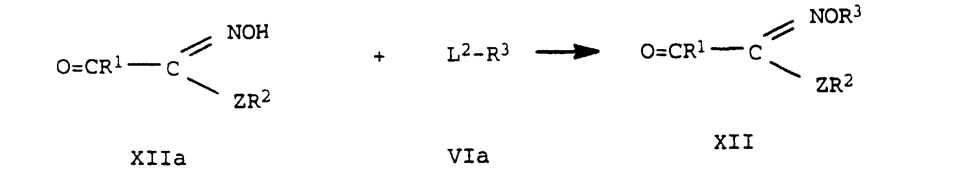

Die Umsetzung erfolgt in an sich bekannter Weise in einem inerten organischen Lösungsmittel gemäß den in Houben-Weyl Bd. 10/4, S. 217f, Houben-Weyl Bd. E 14b, S. 370f bzw. Heterocycles 36, 1027 (1993) beschriebenen Methoden.

Die Verbindungen XIIa sind bekannt (EP-A 056 161, EP-A 051 792, DE-A 21 11 459) oder können gemäß den in der Literatur beschriebenen Verfahren erhalten werden.

Entsprechend können die Verbindungen I auch dadurch erhalten werden, daß man das Benzylhydroxylamin IIa zunächst mit dem Hydroxyiminoderivat XIIa in das entsprechende Benzyloxyiminoderivat der Formel V überführt und V anschließend mit dem nucleophil substituierten Reagens VIa wie vorstehend beschrieben zu I umsetzt.

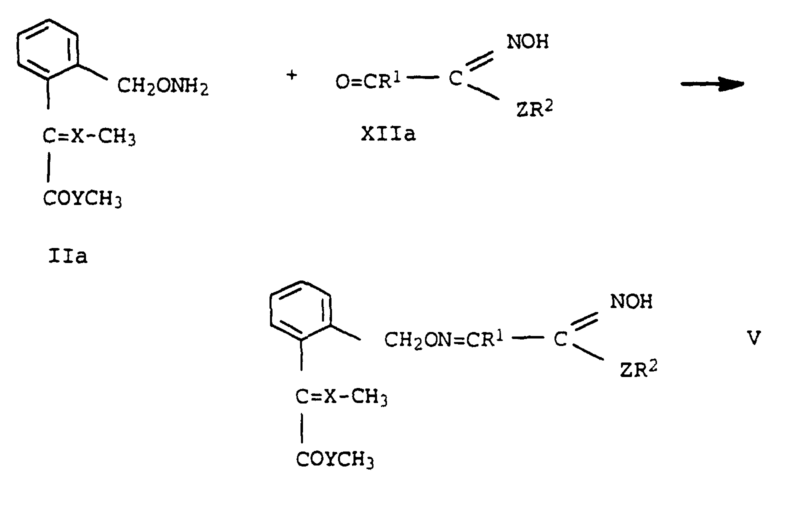

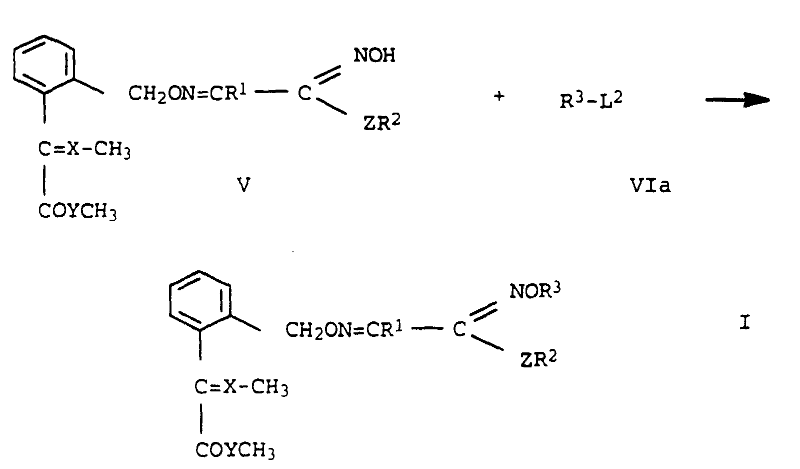

In Analogie zu den vorstehend beschriebenen Verfahren erhält man die Verbindungen VIIIa bzw. VIIIb auch dadurch, daß man ein Benzylhydroxylamin der Formel IIa gemäß den vorstehend beschriebenen Bedingungen mit einem α-Ketosäureester XIIIa bzw. einer α-Ketosäure XIIIb umsetzt.

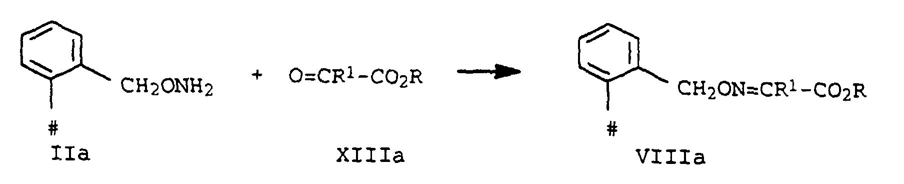

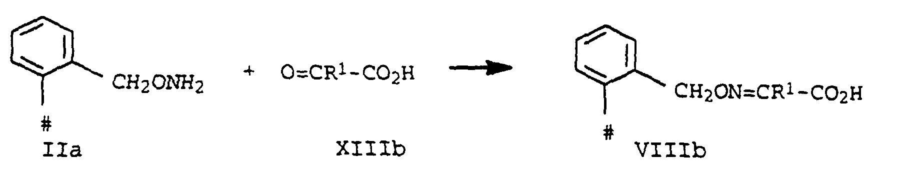

In entsprechender Weise können die Verbindungen Xa und Xb dadurch erhalten werden, daß man ein Benzylhydroxylaminoderivat der Formel IIa mit einer α-Ketohydroxamsäure XIVa bzw. einem α-Ketohydroxamsäurehalogenid XIVb umsetzt.

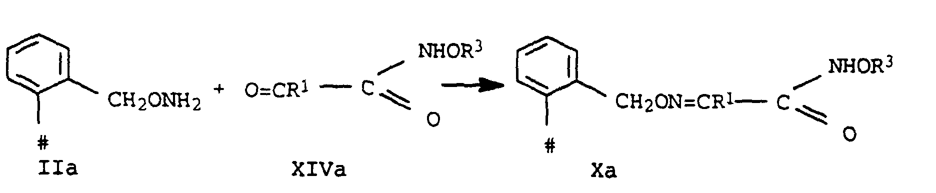

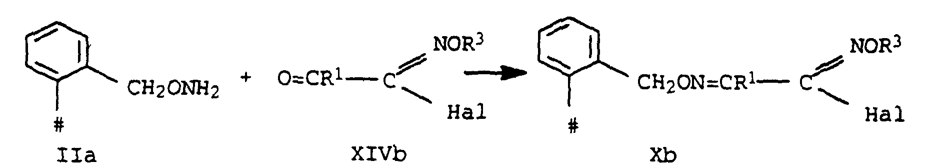

Die Verbindungen XIVb sind bekannt [Arch. Pharm. 310, 30f (1977)] oder können nach den in der Literatur beschriebenen Methoden hergestellt werden.

Des weiteren erhält man die Verbindungen I auch dadurch, daß man Phthalid gemäß den in EP-A 493 711 beschriebenen Methoden mit einer Verbindung der Formel III umsetzt, die so erhaltene Benzoesäure XVa über das entsprechende Halogenid XVb in die Cyanocarbonsäure XVc überführt, welche anschließend im Wege der Pinner-Reaktion [Angew. Chem. 94, 1 (1982)] in den α-Ketoester XVIa überführt und ggf. weiter zum α-Ketoamid XVIb umgesetzt wird [vgl. EP-A 178 826, EP-A 348 766, DE-A 36 23 921, DE-A 37 05 389, Houben-Weyl Bd. E 5, S. 941f].

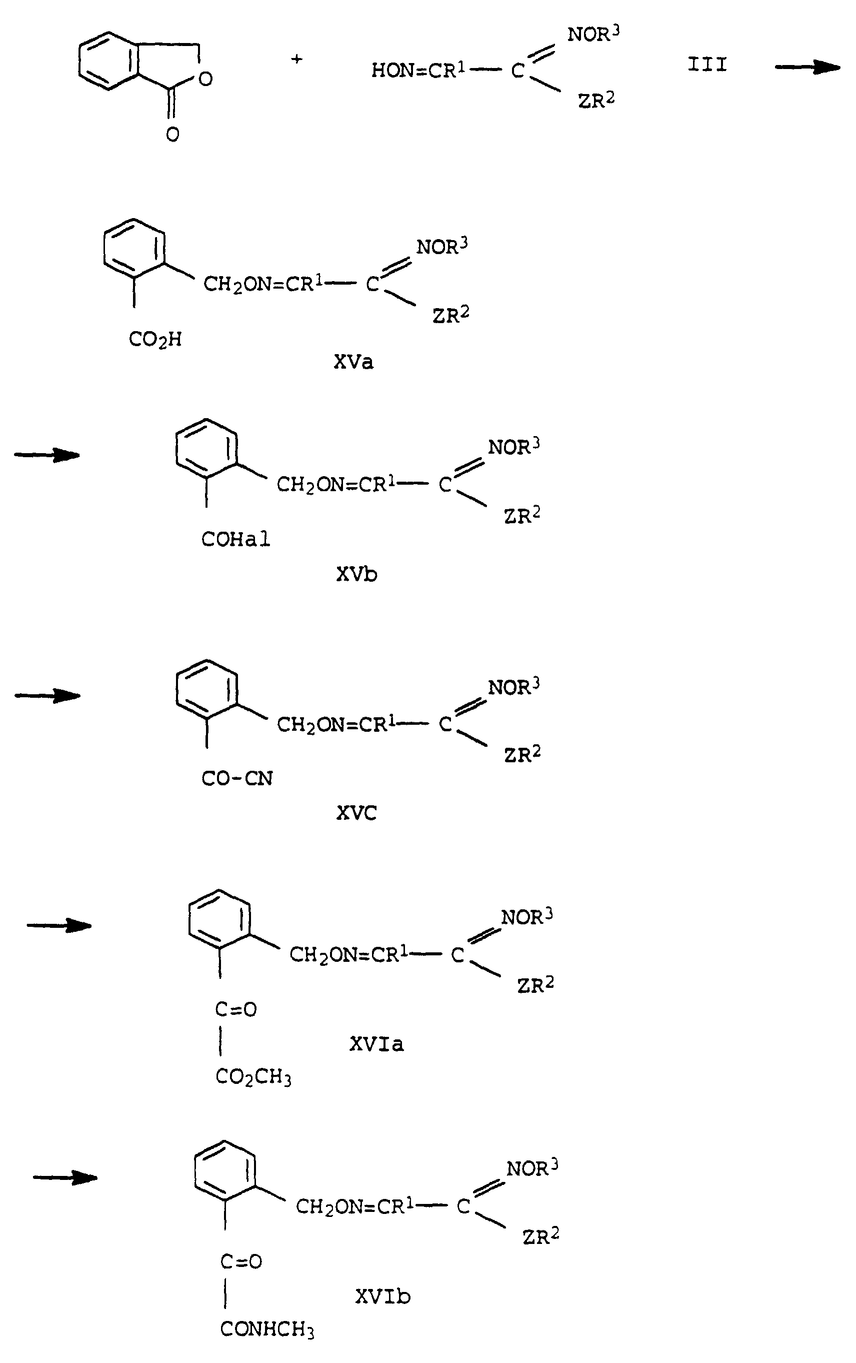

Der α-Ketoester XVIa und das α-Ketoamid XVIb können gemäß üblichen Verfahren in die Verbindungen I überführt werden [vgl. EP-A 178 826, EP-A 513 580, DE-A 36 23 921, EP-A 398 692].

In Abwandlung des vorstehend beschriebenen Verfahrens können die α-Ketoamide XVIb auch durch Umsetzung der Carbonsäurehalogenide XVb mit Methylisocyanat und anschließende Hydrolyse erhalten werden [vgl. EP-A 547 825].

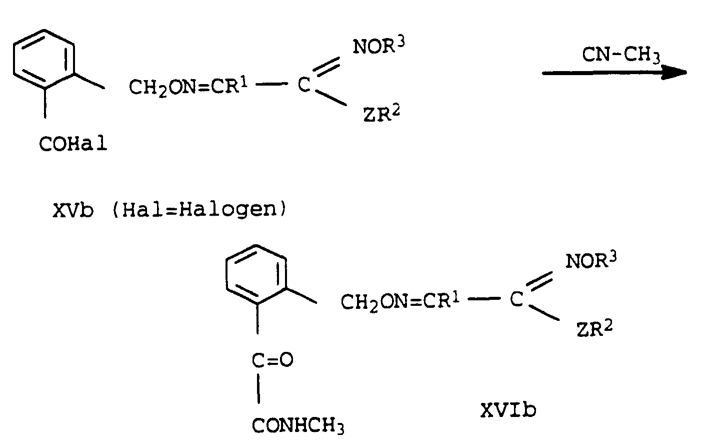

In einer weiteren Variante erhält man die Verbindungen XVIb dadurch, daß man eine ortho-Halogenphenylverbindung nach Metallierung mit Oxalylchlorid in das entsprechende Ketocarbonsäurechlorid überführt, welches anschließen mit Methylamin zu XVIb umgesetzt wird [vgl. J. Org. Chem. 46, 212 (1981); DE-A 40 42 280; Houben-Weyl Bd. E 5, S. 972f].

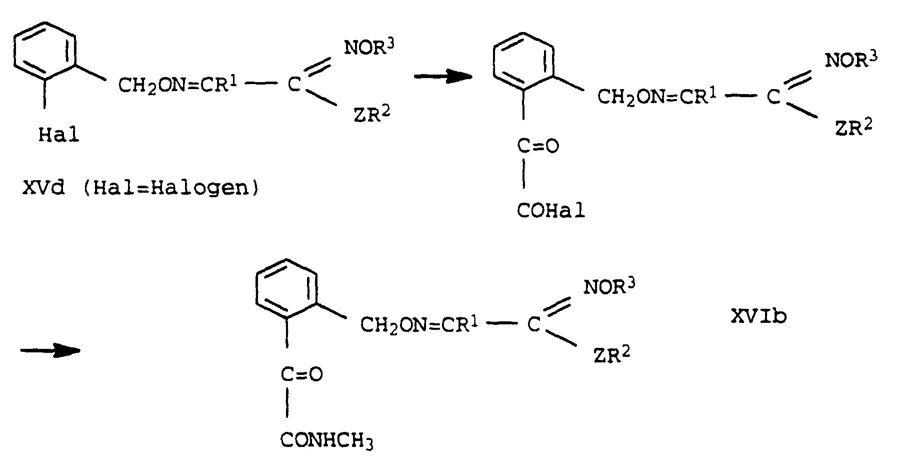

Die Verbindungen I, in denen Y für NH steht, können auch aus den entsprechenden Estern (Y = O) durch Umsetzung mit Methylamin erhalten werden.

Die Verbindungen II sind bekannt [EP-A 251 082, EP-A 400 417, EP-A 463 488, EP-A 477 631, EP-A 513 580, EP-A 585 751] oder können nach den in der Literatur beschriebenen Verfahren synthesisiert werden.

Die Verbindungen I können bei der bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen aufgetrennt werden können.

In Bezug auf die C=X-Doppelbindung werden hinsichtlich ihrer Wirksamkeit die E-Isomere der Verbindungen I bevorzugt (Konfiguration bezogen auf die CH₃ bzw. OCH₃ Gruppe zur COYCH₃ Gruppe).
In Bezug auf die CH₂ON=CR¹-Doppelbindung werden hinsichtlich ihrer Wirksamkeit die *cis*-Isomere der Verbindungen I bevorzugt (Konfiguration bezogen auf die Reste R¹ im Verhältnis zur OCH₂-Gruppierung).

Sofern bei der Synthese Isomerengemische gebildet werden, ist die Trennung in die Einzelverbindungen im allgemeinen jedoch nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, Pilzen und tierischen Schädlingen im behandelten Organismus erfolgen.

Bei der eingangs angegebenen Definition der Verbindungen I wurden Sammelbegriffe verwendet, die allgemein repräsentativ für die folgenden Gruppen stehen:
Halogen: Fluor, Chlor, Brom und Jod;
Alkyl: gesättigte, geradkettige oder verzweigte Kohlenwasserstoffreste mit 1 bis 6 Kohlenstoffatomen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkylamino: eine geradkettige oder verzweigte Alkylgruppe mit 1 bis 6 Kohlenstoffatomen (wie vorstehend genannt), welche über eine Aminogruppe (-NH-) an das Gerüst gebunden ist;
Dialkylamino: eine Aminogruppe, welche zwei voneinander unabhängige, geradkettige oder verzweigte Alkylgruppen mit jeweils 1 bis 6 Kohlenstoffatomen (wie vorstehend genannt) trägt;
Alkylaminocarbonyl: eine Alkylaminogruppe (wie vorstehend genannt), welche über eine Carbonylgruppe (-CO-) an das Gerüst gebunden ist;
Dialkylaminocarbonyl: eine Dialkylaminogruppe (wie vorstehend genannt), welche über eine Carbonylgruppe (-CO-) an das Gerüst gebunden ist;
Alkylcarbonyl: geradkettige oder verzweigte Alkylgruppen mit 1 bis 4 Kohlenstoffatomen (wie vorstehend genannt), welche über eine Carbonylgruppe (-CO-) an das Gerüst gebunden sind;
Alkylsulfonyl: geradkettige oder verzweigte Alkylgruppen mit 1 bis 6 Kohlenstoffatomen (wie vorstehend genannt), welche über eine Sulfonylgruppe (-SO₂-) an das Gerüst gebunden sind;
Alkylsulfoxyl: geradkettige oder verzweigte Alkylgruppen mit 1 bis 6 Kohlenstoffatomen (wie vorstehend genannt), welche über eine Sulfoxylgruppe (......) an das Gerüst gebunden sind;
Halogenalkyl: geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 6 Kohlenstoffatomen (wie vorstehend genannt), welche über ein Sauerstoffatom (-O-) an das Gerüst gebunden sind;
Alkoxycarbonyl: geradkettige oder verzweigte Alkoxygruppen mit 1 bis 6 Kohlenstoffatomen (wie vorstehend genannt), welche über eine Carbonylgruppe (-CO-) an das Gerüst gebunden sind;
Halogenalkoxy: geradkettige oder verzweigte Halogenalkylgruppen mit 1 bis 6 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 6 Kohlenstoffatomen (wie vorstehend genannt), welche über ein Schwefelatom (-S-) an das Gerüst gebunden sind;
Halogenalkylthio: geradkettige oder verzweigte Halogenalkylgruppen mit 1 bis 6 Kohlenstoffatomen (wie vorstehend genannt), welche über ein Schwefelatom (-S-) an das Gerüst gebunden sind;
Alkenyl: ungesättigte, geradkettige oder verzweigte Kohlenwasserstoffreste mit 2 bzw. 3 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkenyloxy: geradkettige oder verzweigte Alkenylgruppen mit 2 bis 6 Kohlenstoffatomen (wie vorstehend genannt), welche über ein Sauerstoffatom an das Gerüst gebunden sind;
Alkinyl: geradkettige oder verzweigte Kohlenwasserstoffgruppen mit 2 bis 6 Kohlenstoffatomen und einer Dreifachbindung in einer beliebigen Position wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: monocyclische Alkylgruppen mit 3 bis 6 Kohlenstoffringgliedern wie Cyclopropyl, Cyclobutyl, Cyclopentyl und Cyclohexyl;
Aryl: ein aromatischer monocyclischer oder polycyclischer Kohlenwasserstoffrest wie Phenyl, Naphthyl und Anthracenyl;
Heteroaryl: ein aromatischer, monocyclischer oder polycyclischer Rest, welcher neben Kohlenstoff noch Heteroatome aus der Gruppe Sauerstoff, Schwefel und Stickstoff als Ringglieder enthalten kann, z.B.
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol -3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Die Angabe *"partiell oder vollständig halogeniert"* soll zum Ausdruck bringen, daß in derart charakterisierten Gruppen die Wasserstoffatome (der Kohlenwasserstoffgruppen) zum Teil oder vollständig durch gleiche oder verschiedene Halogenatome wie vorstehend genannt ersetzt sein können.

Im Hinblick auf ihre biologische Wirkung sind insbesondere Verbindungen I bevorzugt, in denen X NO bedeutet.

Gleichermaßen sind Verbindungen I bevorzugt, in denen X CHO bedeutet.

Daneben sind Verbindungen I bevorzugt, in denen X CH bedeutet.

Außerdem sind Verbindungen I bevorzugt, in denen Y Sauerstoff bedeutet.

Desweiteren sind Verbindungen I bevorzugt, in denen Y NH bedeutet.

Gleichermaßen sind Verbindungen I bevorzugt, in denen R¹ C₁-C₄-Alkyl, insbesondere Methyl, bedeutet.

Daneben sind Verbindungen I bevorzugt, in denen R¹ C₁-C₄-Halogenalkyl, insbesondere Trifluormethyl, bedeutet.

Weiterhin sind Verbindungen I bevorzugt, in denen R² die folgende Bedeutung hat:
C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
   - C₃-C₆-Cycloalkyl, Phenyl oder Oxiranyl, wobei die cyclischen Reste ihrerseits partiell oder vollständig halogeniert sein können und/oder eine bis drei der folgenden Gruppen tragen können: Cyano, C₁-C₄-Alkyl, C₁-C₄-Halogenalkyl, C₁-C₆-Alkoxy, C₁-C₆-Halogenalkoxy, C₁-C₆-Alkoxycarbonyl, C₁-C₆-Alkylthio, C₁-C₆-Halogenalkylthio und Phenyl;
C₃-C₆-Cycloalkyl, wobei diese Gruppen partiell oder vollständig halogeniert sein können und/oder einen bis drei der folgenden Reste tragen können:
   - Cyano, C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio.

Gleichermaßen sind Verbindungen I bevorzugt, in denen R² für unsusbstituiertes oder substituiertes Benzyl oder für Alkyl, Alkenyl oder Alkinyl mit jeweils 3 bis 6 Kohlenstoffatomen steht. Daneben sind Verbindungen I bevorzugt, in denen R² für Benzyl steht, welches partiell oder vollständig halogeniert sein kann und/oder einen bis drei der folgenden Reste tragen kann: Cyano, C₁-C₄-Alkyl, C₁-C₄-Halogenalkyl, C₁-C₄-Alkylcarbonyl, C₁-C₄-Alkoxy, C₁-C₄-Halogenalkoxy, C₁-C₄-Alkoxycarbonyl und Alkylthio.

Außerdem sind Verbindungen I bevorzugt, in denen R² für C₃-C₆-Cycloalkyl steht.

Gleichermaßen sind Verbindungen I bevorzugt, in denen R³ für C₁-C₆-Alkyl (bevorzugt C₁-C₄-Alkyl, insbesondere C₁-C₂-Alkyl), welches partiell oder vollständig halogeniert sein kann, steht. Daneben sind Verbindungen I bevorzugt, in denen R³ für C₂-C₆-Alkenyl (bevorzugt C₃-C₆-Alkenyl, insbesondere C₃-C₄-Alkenyl), welches partiell oder vollständig halogeniert sein kann, steht.

Außerdem sind Verbindungen I bevorzugt, in denen R³ für C₂-C₆-Alkinyl (bevorzugt C₃-C₆-Alkinyl, insbesondere C₃-C₄-Alkinyl), welches partiell oder vollständig halogeniert sein kann, steht.

Die in der folgenden Tabelle genannten Gruppen stellen eine besonders bevorzugte Ausgestaltung des betreffenden Substituenten R² dar.

**Tabelle A**

| Nr. | R² |
|---|---|
| 1 | CH₂CH₃ |
| 2 | CH₂CH₂CH₃ |
| 3 | CH(CH₃)₂ |
| 4 | cyclopropyl |
| 5 | (CH₂)₃CH₃ |
| 6 | CH(CH₃)CH₂CH₃ |
| 7 | CH₂CH(CH₃)₂ |
| 8 | C(CH₃)₃ |
| 9 | cyclobutyl |
| 10 | (CH₂)₄CH₃ |
| 11 | CH(CH₃)(CH₂)₂CH₃ |
| 12 | CH₂CH(CH₃)CH₂CH₃ |
| 13 | (CH₂)₂CH(CH₃)₂ |
| 14 | CH₂C(CH₃)₃ |
| 15 | CH(CH₂CH₃)₂ |
| 16 | C(CH₃)₂CH₂CH₃ |
| 17 | CH(CH₃)CH(CH₃)₂ |
| 18 | cyclopentyl |
| 19 | (CH₂)₅CH₃ |
| 20 | CH(CH₃)(CH₂)₃CH₃ |
| 21 | CH(CH₂CH₃)(CH₂)₂CH₃ |
| 22 | CH₂CH(CH₃)(CH₂)₂CH₃ |
| 23 | (CH₂)₂CH(CH₃)CH₂CH₃ |
| 24 | (CH₂)₃CH(CH₃)₂ |
| 25 | (CH₂)₂C(CH₃)₃ |
| 26 | CH₂CH(CH₂CH₃)₂ |
| 27 | CH(CH₃)CH(CH₃)CH₂CH₃ |
| 28 | CH(CH₃)CH₂CH(CH₃)₂ |
| 29 | CH₂CH(CH₃)CH(CH₃)₂ |
| 30 | CH(CH₃)C(CH₃)₃ |
| 31 | CH(CH₂CH₃)CH(CH₃)₂ |
| 32 | C(CH₃)₂CH₂CH₂CH₃ |
| 33 | CH₂C(CH₃)₂CH₂CH₃ |
| 34 | C(CH₃)₂CH(CH₃)₂ |
| 35 | cyclohexyl |
| 36 | CH₂CN |
| 37 | (CH₂)₂CN |
| 38 | (CH₂)₃CN |
| 39 | (CH₂)₄CN |
| 40 | CH₂NO₂ |
| 41 | (CH₂)₂NO₂ |
| 42 | (CH₂)₃NO₂ |
| 43 | (CH₂)₄NO₂ |
| 44 | (CH₂)₂OH |
| 45 | (CH₂)₃OH |
| 46 | (CH₂)₄OH |
| 47 | (CH₂)₂NH₂ |
| 48 | (CH₂)₃NH₂ |
| 49 | (CH₂)₄NH₂ |
| 50 | (CH₂)₂NHCH₃ |
| 51 | (CH₂)₃NHCH₃ |
| 52 | (CH₂)₄NHCH₃ |
| 53 | (CH₂)₂N(CH₃)₂ |
| 54 | (CH₂)₃N(CH₃)₂ |
| 55 | (CH₂)₄N(CH₃)₂ |
| 56 | (CH₂)₂N(CH₂CH₃)₂ |
| 57 | (CH₂)₃N(CH₂CH₃)₂ |
| 58 | (CH₂)₄N(CH₂CH₃)₂ |
| 59 | (CH₂)₂OCH₃ |
| 60 | (CH₂)₃OCH₃ |
| 61 | (CH₂)₄OCH₃ |
| 62 | (CH₂)₂OCH₂CH₃ |
| 63 | (CH₂)₃OCH₂CH₃ |
| 64 | (CH₂)₄OCH₂CH₃ |
| 65 | (CH₂)₂O(CH₂)₂CH₃ |
| 66 | (CH₂)₃O(CH₂)₂CH₃ |
| 67 | (CH₂)₄O(CH₂)₂CH₃ |
| 68 | (CH₂)₂OCH(CH₃)₂ |
| 69 | (CH₂)₃OCH(CH₃)₂ |
| 70 | (CH₂)₄OCH(CH₃)₂ |
| 71 | (CH₂)₂OC(CH₃)₃ |
| 72 | (CH₂)₃OC(CH₃)₃ |
| 73 | (CH₂)₄OC(CH₃)₃ |
| 74 | (CH₂)₂OCF₃ |
| 75 | (CH₂)₃OCF₃ |
| 76 | (CH₂)₄OCF₃ |
| 77 | (CH₂)₂SCH₃ |
| 78 | (CH₂)₃SCH₃ |
| 79 | (CH₂)₄SCH₃ |
| 80 | (CH₂)₂SOCH₃ |
| 81 | (CH₂)₃SOCH₃ |
| 82 | (CH₂)₄SOCH₃ |
| 83 | (CH₂)₂SO₂CH₃ |
| 84 | (CH₂)₃SO₂CH₃ |
| 85 | (CH₂)₄SO₂CH₃ |
| 86 | CH₂-cyclopropyl |
| 87 | (CH₂)2-cyclopropyl |
| 88 | (CH₂)₃-cyclopropyl |
| 89 | (CH₂)₄-cyclopropyl |
| 90 | CH₂-cyclopentyl |
| 91 | (CH₂)₂-cyclopentyl |
| 92 | (CH₂)₃-cyclopentyl |
| 93 | (CH₂)₄-cyclopentyl |
| 94 | CH₂-cyclohexyl |
| 95 | (CH₂)₂-cyclohexyl |
| 96 | (CH₂)₃-cyclohexyl |
| 97 | (CH₂)₄-cyclohexyl |
| 98 | CHF₂ |
| 99 | CF₃ |
| 100 | CH₂CHF₂ |
| 101 | CH₂CF₃ |
| 102 | CHFCHF₂ |
| 103 | CH₂CH₂F |
| 104 | CHFCH₃ |
| 105 | CHFCF₃ |
| 106 | CF₂CHF₂ |
| 107 | CF₂CHFCF₃ |
| 108 | CH₂CCl₃ |
| 109 | CF₂CHCl₂ |
| 110 | CF₂CHFCl |
| 111 | CF₂CHFBr |
| 112 | CH(CF₃)₂ |
| 113 | CH(CF₃)CH₃ |
| 114 | CH₂CH₂CF₃ |
| 115 | CH₂CHFCH₃ |
| 116 | CH₂CF₂CF₃ |
| 117 | CH₂CH₂CH₂F |
| 118 | CH₂CF₂CF₂CF₃ |
| 119 | CH₂CH₂CHFCH₃ |
| 120 | CH₂CH₂CH₂CH₂F |
| 121 | CH₂CH₂Cl |
| 122 | CH₂CHClCH₃ |
| 123 | CH₂CH₂CH₂Cl |
| 124 | CH₂CH₂CHClCH₃ |
| 125 | CH₂CH₂CH₂CH₂Cl |
| 126 | CH₂CH₂Br |
| 127 | CH₂CHBrCH₃ |
| 128 | CH₂CH₂CH₂Br |
| 129 | CH₂CH₂CHBrCH₃ |
| 130 | CH₂CH₂CH₂CH₂Br |
| 131 | CH₂-C₆H₅ |
| 132 | CH(CH₃)CN |
| 133 | CH(CH₃)CH₂CN |
| 134 | CH₂CH(CH₃)CN |
| 135 | CH(CH₃)CH(CH₃)CN |
| 136 | CH(CH₃)(CH₂)₂CN |
| 137 | CH₂CH(CH₃)CH₂CN |
| 138 | (CH₂)₂CH(CH₃)CN |
| 139 | CH(CH₃)CH(CH₃)CH₂CN |
| 140 | CH(CH₃)CH₂CH(CH₃)CN |
| 141 | CH₂CH(CH₃)CH(CH₃)CN |
| 142 | CH(CH₃)CH(CH₃)CH(CH₃)CN |
| 143 | CH(CH₃)(CH₂)₃CN |
| 144 | CH(CH₃)NO₂ |
| 145 | CH(CH₃)CH₂NO₂ |
| 146 | CH₂CH(CH₃)NO₂ |
| 147 | CH(CH₃)CH(CH₃)NO₂ |
| 148 | CH(CH₃)(CH₂)₂NO₂ |
| 149 | CH₂CH(CH₃)CH₂NO₂ |
| 150 | (CH₂)₂CH(CH₃)NO₂ |
| 151 | CH(CH₃)CH(CH₃)CH₂NO₂ |
| 152 | CH(CH₃)CH₂CH(CH₃)NO₂ |
| 153 | CH₂CH(CH₃)CH(CH₃)NO₂ |
| 154 | CH(CH₃)CH(CH₃)CH(CH₃)NO₂ |
| 155 | CH(CH₃)(CH₂)₃NO₂ |
| 156 | CH(CH₃)CH₂OH |
| 157 | CH₂CH(CH₃)OH |
| 158 | CH(CH₃)CH(CH₃)OH |
| 159 | CH(CH₃)(CH₂)₂OH |
| 160 | CH₂CH(CH₃)CH₂OH |
| 161 | (CH₂)₂CH(CH₃)OH |
| 162 | CH(CH₃)CH(CH₃)CH₂OH |
| 163 | CH(CH₃)CH₂CH(CH₃)OH |
| 164 | CH₂CH(CH₃)CH(CH₃)OH |
| 165 | CH(CH₃)CH(CH₃)CH(CH₃)OH |
| 166 | CH(CH₃)(CH₂)₃OH |
| 167 | CH(CH₃)CH₂OCH₃ |
| 168 | CH₂CH(CH₃)OCH₃ |
| 169 | CH(CH₃)CH(CH₃)OCH₃ |
| 170 | CH(CH₃)(CH₂)₂OCH₃ |
| 171 | CH₂CH(CH₃)CH₂OCH₃ |
| 172 | (CH₂)₂CH(CH₃)OCH₃ |
| 173 | CH(CH₃)CH(CH₃)CH₂OCH₃ |
| 174 | CH(CH₃)CH₂CH(CH₃)OCH₃ |
| 175 | CH₂CH(CH₃)CH(CH₃)OCH₃ |
| 176 | CH(CH₃)CH(CH₃)CH(CH₃)OCH₃ |
| 177 | CH(CH₃)(CH₂)₃OCH₃ |
| 178 | CH(CH₃)CH₂OCH₂CH₃ |
| 179 | CH₂CH(CH₃)OCH₂CH₃ |
| 180 | CH(CH₃)CH(CH₃)OCH₂CH₃ |
| 181 | CH(CH₃)(CH₂)₂OCH₂CH₃ |
| 182 | CH₂CH(CH₃)CH₂OCH₂CH₃ |
| 183 | (CH₂)₂CH(CH₃)OCH₂CH₃ |
| 184 | CH(CH₃)CH(CH₃)CH₂OCH₂CH₃ |
| 185 | CH(CH₃)CH₂CH(CH₃)OCH₂CH₃ |
| 186 | CH₂CH(CH₃)CH(CH₃)OCH₂CH₃ |
| 187 | CH(CH₃)CH(CH₃)CH(CH₃)OCH₂CH₃ |
| 188 | CH(CH₃)(CH₂)₃OCH₂CH₃ |
| 189 | CH(CH₃)CH₂O(CH₂)₂CH₃ |
| 190 | CH₂CH(CH₃)O(CH₂)₂CH₃ |
| 191 | CH(CH₃)CH(CH₃)O(CH₂)₂CH₃ |
| 192 | CH(CH₃)(CH₂)₂O(CH₂)₂CH₃ |
| 193 | CH₂CH(CH₃)CH₂O(CH₂)₂CH₃ |
| 194 | (CH₂)₂CH(CH₃)O(CH₂)₂CH₃ |
| 195 | CH(CH₃)CH(CH₃)CH₂O(CH₂)₂CH₃ |
| 196 | CH(CH₃)CH₂CH(CH₃)O(CH₂)₂CH₃ |
| 197 | CH₂CH(CH₃)CH(CH₃)O(CH₂)₂CH₃ |
| 198 | CH(CH₃)CH(CH₃)CH(CH₃)O(CH₂)₂CH₃ |
| 199 | CH(CH₃)(CH₂)₃O(CH₂)₂CH₃ |
| 200 | CH(CH₃)CH₂OCH(CH₃)₂ |
| 201 | CH₂CH(CH₃)OCH(CH₃)₂ |
| 202 | CH(CH₃)CH(CH₃)OCH(CH₃)₂ |
| 203 | CH(CH₃)(CH₂)₂OCH(CH₃)₂ |
| 204 | CH₂CH(CH₃)CH₂OCH(CH₃)₂ |
| 205 | (CH₂)₂CH(CH₃)OCH(CH₃)₂ |
| 206 | CH(CH₃)CH(CH₃)CH₂OCH(CH₃)₂ |
| 207 | CH(CH₃)CH₂CH(CH₃)OCH(CH₃)₂ |
| 208 | CH₂CH(CH₃)CH(CH₃)OCH(CH₃)₂ |
| 209 | CH(CH₃)CH(CH₃)CH(CH₃)OCH(CH₃)₂ |
| 210 | CH(CH₃)(CH₂)₃OCH(CH₃)₂ |
| 211 | CH(CH₃)CH₂OC(CH₃)₃ |
| 212 | CH₂CH(CH₃)OC(CH₃)₃ |
| 213 | CH(CH₃)CH(CH₃)OC(CH₃)₃ |
| 214 | CH(CH₃)(CH₂)₂OC(CH₃)₃ |
| 215 | CH₂CH(CH₃)CH₂OC(CH₃)₃ |
| 216 | (CH₂)₂CH(CH₃)OC(CH₃)₃ |
| 217 | CH(CH₃)CH(CH₃)CH₂OC(CH₃)₃ |
| 218 | CH(CH₃)CH₂CH(CH₃)OC(CH₃)₃ |
| 219 | CH₂CH(CH₃)CH(CH₃)OC(CH₃)₃ |
| 220 | CH(CH₃)CH(CH₃)CH(CH₃)OC(CH₃)₃ |
| 221 | CH(CH₃)(CH₂)₃OC(CH₃)₃ |
| 222 | CH(CH₃)CH₂OCF₃ |
| 223 | CH₂CH(CH₃)OCF₃ |
| 224 | CH(CH₃)CH(CH₃)OCF₃ |
| 225 | CH(CH₃)(CH₂)₂OCF₃ |
| 226 | CH₂CH(CH₃)CH₂OCF₃ |
| 227 | (CH₂)₂CH(CH₃)OCF₃ |
| 228 | CH(CH₃)CH(CH₃)CH₂OCF₃ |
| 229 | CH(CH₃)CH₂CH(CH₃)OCF₃ |
| 230 | CH₂CH(CH₃)CH(CH₃)OCF₃ |
| 231 | CH(CH₃)CH(CH₃)CH(CH₃)OCF₃ |
| 232 | CH(CH₃)(CH₂)₃OCF₃ |
| 233 | CH(CH₃)CH₂SCH₃ |
| 234 | CH₂CH(CH₃)SCH₃ |
| 235 | CH(CH₃)CH(CH₃)SCH₃ |
| 236 | CH(CH₃)(CH₂)₂SCH₃ |
| 237 | CH₂CH(CH₃)CH₂SCH₃ |
| 238 | (CH₂)₂CH(CH₃)SCH₃ |
| 239 | CH(CH₃)CH(CH₃)CH₂SCH₃ |
| 240 | CH(CH₃)CH₂CH(CH₃)SCH₃ |
| 241 | CH₂CH(CH₃)CH(CH₃)SCH₃ |
| 242 | CH(CH₃)CH(CH₃)CH(CH₃)SCH₃ |
| 243 | CH(CH₃)(CH₂)₃SCH₃ |
| 244 | CH(CH₃)CH₂SOCH₃ |
| 245 | CH₂CH(CH₃)SOCH₃ |
| 246 | CH(CH₃)CH(CH₃)SOCH₃ |
| 247 | CH(CH₃)(CH₂)₂SOCH₃ |
| 248 | CH₂CH(CH₃)CH₂SOCH₃ |
| 249 | (CH₂)₂CH(CH₃)SOCH₃ |
| 250 | CH(CH₃)CH(CH₃)CH₂SOCH₃ |
| 251 | CH(CH₃)CH₂CH(CH₃)SOCH₃ |
| 252 | CH₂CH(CH₃)CH(CH₃)SOCH₃ |
| 253 | CH(CH₃)CH(CH₃)CH(CH₃)SOCH₃ |
| 254 | CH(CH₃)(CH₂)₃SOCH₃ |
| 255 | CH(CH₃)CH₂SO₂CH₃ |
| 256 | CH₂CH(CH₃)SO₂CH₃ |
| 257 | CH(CH₃)CH(CH₃)SO₂CH₃ |
| 258 | CH(CH₃)(CH₂)₂SO₂CH₃ |
| 259 | CH₂CH(CH₃)CH₂SO₂CH₃ |
| 260 | (CH₂)₂CH(CH₃)SO₂CH₃ |
| 261 | CH(CH₃)CH(CH₃)CH₂SO₂CH₃ |
| 262 | CH(CH₃)CH₂CH(CH₃)SO₂CH₃ |
| 263 | CH₂CH(CH₃)CH(CH₃)SO₂CH₃ |
| 264 | CH(CH₃)CH(CH₃)CH(CH₃)SO₂CH₃ |
| 265 | CH(CH₃)(CH₂)₃SO₂CH₃ |
| 266 | CH(CH₃)-cyclopropyl |
| 267 | CH(CH₃)CH₂-cyclopropyl |
| 268 | CH₂CH(CH₃)-cyclopropyl |
| 269 | CH(CH₃)CH(CH₃)-cyclopropyl |
| 270 | CH(CH₃)(CH₂)₂-cyclopropyl |
| 271 | CH₂CH(CH₃)CH₂-cyclopropyl |
| 272 | (CH₂)₂CH(CH₃)-cyclopropyl |
| 273 | CH(CH₃)CH(CH₃)CH₂-cyclopropyl |
| 274 | CH(CH₃)CH₂CH(CH₃)-cyclopropyl |
| 275 | CH₂CH(CH₃)CH(CH₃)-cyclopropyl |
| 276 | CH(CH₃)CH(CH₃)CH(CH₃)-cyclopropyl |
| 277 | CH(CH₃)(CH₂)₃-cyclopropyl |
| 278 | CH(CH₃)-cyclopentyl |
| 279 | CH(CH₃)CH₂-cyclopentyl |
| 280 | CH₂CH(CH₃)-cyclopentyl |
| 281 | CH(CH₃)CH(CH₃)-cyclopentyl |
| 282 | CH(CH₃)(CH₂)₂-cyclopentyl |
| 283 | CH₂CH(CH₃)CH₂-cyclopentyl |
| 284 | (CH₂)₂CH(CH₃)-cyclopentyl |
| 285 | CH(CH₃)CH(CH₃)CH₂-cyclopentyl |
| 286 | CH(CH₃)CH₂CH(CH₃)-cyclopentyl |
| 287 | CH₂CH(CH₃)CH(CH₃)-cyclopentyl |
| 288 | CH(CH₃)CH(CH₃)CH(CH₃)-cyclopentyl |
| 289 | CH(CH₃)(CH₂)₃-cyclopentyl |
| 290 | CH(CH₃)-cyclohexyl |
| 291 | CH(CH₃)CH₂-cyclohexyl |
| 292 | CH₂CH(CH₃)-cyclohexyl |
| 293 | CH(CH₃)CH(CH₃)-cyclohexyl |
| 294 | CH(CH₃)(CH₂)₂-cyclohexyl |
| 295 | CH₂CH(CH₃)CH₂-cyclohexyl |
| 296 | (CH₂)₂CH(CH₃)-cyclohexyl |
| 297 | CH(CH₃)CH(CH₃)CH₂-cyclohexyl |
| 298 | CH(CH₃)CH₂CH(CH₃)-cyclohexyl |
| 299 | CH₂CH(CH₃)CH(CH₃)-cyclohexyl |
| 300 | CH(CH₃)CH(CH₃)CH(CH₃)-cyclohexyl |
| 301 | CH(CH₃)(CH₂)₃-cyclohexyl |
| 302 | CH(CH₃)CHF₂ |
| 303 | CF(CH₃)CHF₂ |
| 304 | CH(CH₃)CH₂F |
| 305 | CF(CH₃)CH₃ |
| 306 | CF(CH₃)CF₃ |
| 307 | CH(CH₃)CCl₃ |
| 308 | CH(CH₃)CH₂CF₃ |
| 309 | CH₂CH(CH₃)CF₃ |
| 310 | CH(CH₃)CH(CH₃)CF₃ |
| 311 | CH(CH₃)CF₂CF₃ |
| 312 | CH(CH₃)-phenyl |
| 313 | CH(CH₃)CH₂-phenyl |
| 314 | CH₂CH(CH₃)-phenyl |
| 315 | CH(CH₃)CH(CH₃)-Phenyl |
| 316 | CH(CH₃)(CH₂)₂-phenyl |
| 317 | CH₂CH(CH₃)CH₂-phenyl |
| 318 | (CH₂)₂CH(CH₃)-phenyl |
| 319 | CH(CH₃)CH(CH₃)CH₂-phenyl |
| 320 | CH(CH₃)CH₂CH(CH₃)-phenyl |
| 321 | CH₂CH(CH₃)CH(CH₃)-phenyl |
| 322 | CH(CH₃)CH(CH₃)CH(CH₃)-phenyl |
| 323 | CH(CH₃)(CH₂)₃-phenyl |
| 324 | 2-F-C₆H₄-CH₂ |
| 325 | 3-F-C₆H₄-CH₂ |
| 326 | 4-F-C₆H₄-CH₂ |
| 327 | 2,3-F₂-C₆H₃-CH₂ |
| 328 | 2,4-F₂-C₆H₃-CH₂ |
| 329 | 2,5-F₂-C₆H₃-CH₂ |
| 330 | 2,6-F₂-C₆H₃-CH₂ |
| 331 | 3,4-F₂-C₆H₃-CH₂ |
| 332 | 3,5-F₂-C₆H₃-CH₂ |
| 333 | 2-Cl-C₆H₄-CH₂ |
| 334 | 3-Cl-C₆H₄-CH₂ |
| 335 | 4-Cl-C₆H₄-CH₂ |
| 336 | 2,3-Cl₂-C₆H₃-CH₂ |
| 337 | 2,4-Cl₂-C₆H₃-CH₂ |
| 338 | 2,5-Cl₂-C₆H₃-CH₂ |
| 339 | 2,6-Cl₂-C₆H₃-CH₂ |
| 340 | 3,4-Cl₂-C₆H₃-CH₂ |
| 341 | 3,5-Cl₂-C₆H₃-CH₂ |
| 342 | 2,3,4-Cl₃-C₆H₂-CH₂ |
| 343 | 2,3,5-Cl₃-C₆H₂-CH₂ |
| 344 | 2,3,6-Cl₃-C₆H₂-CH₂ |
| 345 | 2,4,5-Cl₃-C₆H₂-CH₂ |
| 346 | 2,4,6-Cl₃-C₆H₂-CH₂ |
| 347 | 3,4,5-Cl₃-C₆H₂-CH₂ |
| 348 | 2-Br-C₆H₄-CH₂ |
| 349 | 3-Br-C₆H₄-CH₂ |
| 350 | 4-Br-C₆H₄-CH₂ |
| 351 | 2,3-Br₂-C₆H₃-CH₂ |
| 352 | 2,4-Br₂-C₆H₃-CH₂ |
| 353 | 2,5-Br₂-C₆H₃-CH₂ |
| 354 | 2,6-Br₂-C₆H₃-CH₂ |
| 355 | 3,4-Br₂-C₆H₃-CH₂ |
| 356 | 3,5-Br₂-C₆H₃-CH₂ |
| 357 | 2-F, 3-Cl-C₆H₃-CH₂ |
| 358 | 2-F, 4-Cl-C₆H₃-CH₂ |
| 359 | 2-F, 5-Cl-C₆H₃-CH₂ |
| 360 | 2-F, 3-Br-C₆H₃-CH₂ |
| 361 | 2-F, 4-Br-C₆H₃-CH₂ |
| 362 | 2-F, 5-Br-C₆H₃-CH₂ |
| 363 | 2-Cl, 3-F-C₆H₃-CH₂ |
| 364 | 2-Cl, 4-F-C₆H₃-CH₂ |
| 365 | 2-Cl, 5-F-C₆H₃-CH₂ |
| 366 | 2-Cl, 3-Br-C₆H₃-CH₂ |
| 367 | 2-Cl, 4-Br-C₆H₃-CH₂ |
| 368 | 2-Cl, 5-Br-C₆H₃-CH₂ |
| 369 | 2-Br, 3-F-C₆H₃-CH₂ |
| 370 | 2-Br, 4-F-C₆H₃-CH₂ |
| 371 | 2-Br, 5-F-C₆H₃-CH₂ |
| 372 | 2-Br, 3-Cl-C₆H₃-CH₂ |
| 373 | 2-Br, 4-Cl-C₆H₃-CH₂ |
| 374 | 2-Br, 5-Cl-C₆H₃-CH₂ |
| 375 | 4-Cl, 3,5-Br₂-C₆H₂-CH₂ |
| 376 | 2-CN-C₆H₄-CH₂ |
| 377 | 3-CN-C₆H₄-CH₂ |
| 378 | 4-CN-C₆H₄-CH₂ |
| 379 | 2-NO₂-C₆H₄-CH₂ |
| 380 | 3-NO₂-C₆H₄-CH₂ |
| 381 | 4-NO₂-C₆H₄-CH₂ |
| 382 | 2-CH₃-C₆H₄-CH₂ |
| 383 | 3-CH₃-C₆H₄-CH₂ |
| 384 | 4-CH₃-C₆H₄-CH₂ |
| 385 | 2,3-(CH₃)₂-C₆H₃-CH₂ |
| 386 | 2,4-(CH₃)₂-C₆H₃-CH₂ |
| 387 | 2,5-(CH₃)₂-C₆H₃-CH₂ |
| 388 | 2,6-(CH₃)₂-C₆H₃-CH₂ |
| 389 | 3,4-(CH₃)₂-C₆H₃-CH₂ |
| 390 | 3,5-(CH₃)₂-C₆H₃-CH₂ |
| 391 | 2-CH₂CH₃-C₆H₄-CH₂ |
| 392 | 3-CH₂CH₃-C₆H₄-CH₂ |
| 393 | 4-CH₂CH₃-C₆H₄-CH₂ |
| 394 | 2-CH(CH₃)₂-C₆H₄-CH₂ |
| 395 | 3-CH(CH₃)₂-C₆H₄-CH₂ |
| 396 | 4-CH(CH₃)₂-C₆H₄-CH₂ |
| 397 | 3-C(CH₃)₃-C₆H₄-CH₂ |
| 398 | 4-C(CH₃)₃-C₆H₄-CH₂ |
| 399 | 2-C₆H₅-C₆H₄-CH₂ |
| 400 | 3-C₆H₅-C₆H₄-CH₂ |
| 401 | 4-C₆H₅-C₆H₄-CH₂ |
| 402 | 2-OCH₃-C₆H₄-CH₂ |
| 403 | 3-OCH₃-C₆H₄-CH₂ |
| 404 | 4-OCH₃-C₆H₄-CH₂ |
| 405 | 2,3-(OCH₃)₂-C₆H₃-CH₂ |
| 406 | 2,4-(OCH₃)₂-C₆H₃-CH₂ |
| 407 | 2,5-(OCH₃)₂-C₆H₃-CH₂ |
| 408 | 2,6-(OCH₃)₂-C₆H₃-CH₂ |
| 409 | 3,4-(OCH₃)₂-C₆H₃-CH₂ |
| 410 | 3,5-(OCH₃)₂-C₆H₃-CH₂ |
| 411 | 3,4,5-(OCH₃)₃-C₆H₂-CH₂ |
| 412 | 2-OCH₂CH₃-C₆H₄-CH₂ |
| 413 | 3-OCH₂CH₃-C₆H₄-CH₂ |
| 414 | 4-OCH₂CH₃-C₆H₄-CH₂ |
| 415 | 2-O(CH₂)₂CH₃-C₆H₄-CH₂ |
| 416 | 3-O(CH₂)₂CH₃-C₆H₄-CH₂ |
| 417 | 4-O(CH₂)₂CH₃-C₆H₄-CH₂ |
| 418 | 2-OCH(CH₃)₂-C₆H₄-CH₂ |
| 419 | 3-OCH(CH₃)₂-C₆H₄-CH₂ |
| 420 | 4-OCH(CH₃)₂-C₆H₄-CH₂ |
| 421 | 3-OC(CH₃)₃-C₆H₄-CH₂ |
| 422 | 4-OC(CH₃)₃-C₆H₄-CH₂ |
| 423 | 2-OCH₂CH=CH₂-C₆H₄-CH₂ |
| 424 | 3-OCH₂CH=CH₂-C₆H₄-CH₂ |
| 425 | 4-OCH₂CH=CH₂-C₆H₄-CH₂ |
| 426 | 2-CF₃-C₆H₄-CH₂ |
| 427 | 3-CF₃-C₆H₄-CH₂ |
| 428 | 4-CF₃-C₆H₄-CH₂ |
| 429 | 2-CO₂CH₃-C₆H₄-CH₂ |
| 430 | 3-CO₂CH₃-C₆H₄-CH₂ |
| 431 | 4-CO₂CH₃-C₆H₄-CH₂ |
| 432 | 2-CO₂CH₂CH₃-C₆H₄-CH₂ |
| 433 | 3-CO₂CH₂CH₃-C₆H₄-CH₂ |
| 434 | 4-CO₂CH₂CH₃-C₆H₄-CH₂ |
| 435 | 2-CONH₂-C₆H₄-CH₂ |
| 436 | 3-CONH₂-C₆H₄-CH₂ |
| 437 | 4-CONH₂-C₆H₄-CH₂ |
| 438 | 2-CON(CH₃)₂-C₆H₄-CH₂ |
| 439 | 3-CON(CH₃)₂-C₆H₄-CH₂ |
| 440 | 4-CON(CH₃)₂-C₆H₄-CH₂ |
| 441 | 2-CONHCH₃-C₆H₄-CH₂ |
| 442 | 3-CONHCH₃-C₆H₄-CH₂ |
| 443 | 4-CONHCH₃-C₆H₄-CH₂ |
| 444 | 2-NH₂-C₆H₄-CH₂ |
| 445 | 3-NH₂-C₆H₄-CH₂ |
| 446 | 4-NH₂-C₆H₄-CH₂ |
| 447 | 2-N(CH₃)₂-C₆H₄-CH₂ |
| 448 | 3-N(CH₃)₂-C₆H₄-CH₂ |
| 449 | 4-N(CH₃)₂-C₆H₄-CH₂ |
| 450 | 2-NHCH₃-C₆H₄-CH₂ |
| 451 | 3-NHCH₃-C₆H₄-CH₂ |
| 452 | 4-NHCH₃-C₆H₄-CH₂ |
| 453 | 2-CSNH₂-C₆H₄-CH₂ |
| 454 | 3-CSNH₂-C₆H₄-CH₂ |
| 455 | 4-CSNH₂-C₆H₄-CH₂ |
| 456 | 2-SCH₃-C₆H₄-CH₂ |
| 457 | 3-SCH₃-C₆H₄-CH₂ |
| 458 | 4-SCH₃-C₆H₄-CH₂ |
| 459 | 2-SOCH₃-C₆H₄-CH₂ |
| 460 | 3-SOCH₃-C₆H₄-CH₂ |
| 461 | 4-SOCH₃-C₆H₄-CH₂ |
| 462 | 2-SO₂CH₃-C₆H₄-CH₂ |
| 463 | 3-SO₂CH₃-C₆H₄-CH₂ |
| 464 | 4-SO₂CH₃-C₆H₄-CH₂ |
| 465 | 2-OCF₃-C₆H₄-CH₂ |
| 466 | 3-OCF₃-C₆H₄-CH₂ |
| 467 | 4-OCF₃-C₆H₄-CH₂ |
| 468 | 2-OCHF₂-C₆H₄-CH₂ |
| 469 | 3-OCHF₂-C₆H₄-CH₂ |
| 470 | 4-OCHF₂-C₆H₄-CH₂ |
| 471 | 3-CF₃, 4-OCF₃-C₆H₃-CH₂ |
| 472 | 2-CH₂CH₂F-C₆H₄-CH₂ |
| 473 | 3-CH₂CH₂F-C₆H₄-CH₂ |
| 474 | 4-CH₂CH₂F-C₆H₄-CH₂ |
| 475 | 2-CH₂CF₃-C₆H₄-CH₂ |
| 476 | 3-CH₂CF₃-C₆H₄-CH₂ |
| 477 | 4-CH₂CF₃-C₆H₄-CH₂ |
| 478 | 2-CF₂CHF₂-C₆H₄-CH₂ |
| 479 | 3-CF₂CHF₂-C₆H₄-CH₂ |
| 480 | 4-CF₂CHF₂-C₆H₄-CH₂ |
| 481 | 2-CHF₂-C₆H₄-CH₂ |
| 482 | 3-CHF₂-C₆H₄-CH₂ |
| 483 | 4-CHF₂-C₆H₄-CH₂ |
| 484 | naphthalin-1-yl-CH₂ |
| 485 | naphthalin-2-yl-CH₂ |
| 486 | pyridin-2-yl-CH₂ |
| 487 | pyridin-3-yl-CH₂ |
| 488 | pyridin-4-yl-CH₂ |
| 489 | 5-CH₃-pyridin-2-yl-CH₂ |
| 490 | 6-CH₃-pyridin-2-yl-CH₂ |
| 491 | 5-CH₃-pyridin-3-yl-CH₂ |
| 492 | 6-CH₃-pyridin-3-yl-CH₂ |
| 493 | 5-OCH₃-pyridin-2-yl-CH₂ |
| 494 | 6-OCH₃-pyridin-2-yl-CH₂ |
| 495 | 5-OCH₃-pyridin-3-yl-CH₂ |
| 496 | 6-OCH₃-pyridin-3-yl-CH₂ |
| 497 | 4-Cl-pyridin-2-yl-CH₂ |
| 498 | 5-Cl-pyridin-2-yl-CH₂ |
| 499 | 6-Cl-pyridin-2-yl-CH₂ |
| 500 | 2-Cl-pyridin-3-yl-CH₂ |
| 501 | 5-Cl-pyridin-3-yl-CH₂ |
| 502 | 6-Cl-pyridin-3-yl-CH₂ |
| 503 | 2-Cl-pyridin-4-yl-CH₂ |
| 504 | 3,5-Cl-pyridin-2-yl-CH₂ |
| 505 | pyrimidin-2-yl-CH₂ |
| 506 | 4-Cl-pyrimidin-2-yl-CH₂ |
| 507 | 5-Cl-pyrimidin-2-yl-CH₂ |
| 508 | 4-CH₃-pyrimidin-2-yl-CH₂ |
| 509 | 5-CH₃-pyrimidin-2-yl-CH₂ |
| 510 | 4-OCH₃-pyrimidin-2-yl-CH₂ |
| 511 | 5-OCH₃-pyrimidin-2-yl-CH₂ |
| 512 | 4-OCH₂CH₃-pyrimidin-2-yl-CH₂ |
| 513 | 5-OCH₂CH₃-pyrimidin-2-yl-CH₂ |
| 514 | pyrimidin-4-yl-CH₂ |
| 515 | 2-Cl-pyrimidin-4-yl-CH₂ |
| 516 | 6-Cl-pyrimidin-4-yl-CH₂ |
| 517 | 2,6-Cl₂-pyrimidin-4-yl-CH₂ |
| 518 | 2-CH₃-pyrimidin-4-yl-CH₂ |
| 519 | 6-CH₃-pyrimidin-4-yl-CH₂ |
| 520 | 2-OCH₃-pyrimidin-4-yl-CH₂ |
| 521 | 6-OCH₃-pyrimidin-4-yl-CH₂ |
| 522 | 2-OCH₂CH₃-pyrimidin-4-yl-CH₂ |
| 523 | 6-OCH₂CH₃-pyrimidin-4-yl-CH₂ |
| 524 | pyrimidin-5-yl-CH₂ |
| 525 | 2-Cl-pyrimidin-5-yl-CH₂ |
| 526 | 2-CH₃-pyrimidin-5-yl-CH₂ |
| 527 | 2-OCH₃-pyrimidin-5-yl-CH₂ |
| 528 | 2-OCH₂CH₃-pyrimidin-5-yl-CH₂ |
| 529 | furan-2-yl-CH₂ |
| 530 | 4-Br-furan-2-yl-CH₂ |
| 531 | 4-Cl-furan-2-yl-CH₂ |
| 532 | 4-CN-furan-2-yl-CH₂ |
| 533 | 4-CH₃-furan-2-yl-CH₂ |
| 534 | 5-Br-furan-2-yl-CH₂ |
| 535 | 5-Cl-furan-2-yl-CH₂ |
| 536 | 5-CN-furan-2-yl-CH₂ |
| 537 | 5-CH₃-furan-2-yl-CH₂ |
| 538 | furan-3-yl-CH₂ |
| 539 | 5-Br-furan-3-yl-CH₂ |
| 540 | 5-Cl-furan-3-yl-CH₂ |
| 541 | 5-CN-furan-3-yl-CH₂ |
| 542 | 5-CH₃-furan-3-yl-CH₂ |
| 543 | thien-2-yl-CH₂ |
| 544 | 4-Br-thien-2-yl-CH₂ |
| 545 | 4-Cl-thien-2-yl-CH₂ |
| 546 | 4-CN-thien-2-yl-CH₂ |
| 547 | 4-CH₃-thien-2-yl-CH₂ |
| 548 | 5-Br-thien-2-yl-CH₂ |
| 549 | 5-Cl-thien-2-yl-CH₂ |
| 550 | 5-CN-thien-2-yl-CH₂ |
| 551 | 5-CH₃-thien-2-yl-CH₂ |
| 552 | thien-3-yl-CH₂ |
| 553 | 5-Br-thien-3-yl-CH₂ |
| 554 | 5-Cl-thien-3-yl-CH₂ |
| 555 | 5-CN-thien-3-yl-CH₂ |
| 556 | 5-CH₃-thien-3-yl-CH₂ |
| 557 | oxazol-2-yl-CH₂ |
| 558 | 4-Br-oxazol-2-yl-CH₂ |
| 559 | 4-Cl-oxazol-2-yl-CH₂ |
| 560 | 4-CN-oxazol-2-yl-CH₂ |
| 561 | 4-CH₃-oxazol-2-yl-CH₂ |
| 562 | 5-Br-oxazol-2-yl-CH₂ |
| 563 | 5-Cl-oxazol-2-yl-CH₂ |
| 564 | 5-CN-oxazol-2-yl-CH₂ |
| 565 | 5-CH₃-oxazol-2-yl-CH₂ |
| 566 | oxazol-4-yl-CH₂ |
| 567 | 2-Br-oxazol-4-yl-CH₂ |
| 568 | 2-Cl-oxazol-4-yl-CH₂ |
| 569 | 2-CN-oxazol-4-yl-CH₂ |
| 570 | 2-CH₃-oxazol-4-yl-CH₂ |
| 571 | 2-C₆H₅-oxazol-4-yl-CH₂ |
| 572 | 5-Br-oxazol-4-yl-CH₂ |
| 573 | 5-Cl-oxazol-4-yl-CH₂ |
| 574 | 5-CN-oxazol-4-yl-CH₂ |
| 575 | 5-CH₃-oxazol-4-yl-CH₂ |
| 576 | oxazol-5-yl-CH₂ |
| 577 | 4-Br-oxazol-5-yl-CH₂ |
| 578 | 4-Cl-oxazol-5-yl-CH₂ |
| 579 | 4-CN-oxazol-5-yl-CH₂ |
| 580 | 4-CH₃-oxazol-5-yl-CH₂ |
| 581 | 2-Br-oxazol-5-yl-CH₂ |
| 582 | 2-Cl-oxazol-5-yl-CH₂ |
| 583 | 2-CN-Oxazol-5-yl-CH₂ |
| 584 | 2-CH₃-oxazol-5-yl-CH₂ |
| 585 | isoxazol-3-yl-CH₂ |
| 586 | 4-Br-isoxazol-3-yl-CH₂ |
| 587 | 4-Cl-isoxazol-3-yl-CH₂ |
| 588 | 4-CN-isoxazol-3-yl-CH₂ |
| 589 | 4-CH₃-isoxazol-3-yl-CH₂ |
| 590 | 5-Br-isoxazol-3-yl-CH₂ |
| 591 | 5-Cl-isoxazol-3-yl-CH₂ |
| 592 | 5-CN-isoxazol-3-yl-CH₂ |
| 593 | 5-CH₃-isoxazol-3-yl-CH₂ |
| 594 | isoxazol-4-yl-CH₂ |
| 595 | 3-Br-isoxazol-4-yl-CH₂ |
| 596 | 3-Cl-isoxazol-4-yl-CH₂ |
| 597 | 3-CN-isoxazol-4-yl-CH₂ |
| 598 | 3-CH₃-isoxazol-4-yl-CH₂ |
| 599 | 5-Br-isoxazol-4-yl-CH₂ |
| 600 | 5-Cl-isoxazol-4-yl-CH₂ |
| 601 | 5-CN-isoxazol-4-yl-CH₂ |
| 602 | 5-CH₃-isoxazol-4-yl-CH₂ |
| 603 | 3,5-(CH₃)₂-isoxazol-4-yl-CH₂ |
| 604 | isoxazol-5-yl-CH₂ |
| 605 | 3-Br-isoxazol-5-yl-CH₂ |
| 606 | 3-Cl-isoxazol-5-yl-CH₂ |
| 607 | 3-CN-isoxazol-5-yl-CH₂ |
| 608 | 3-CH₃-isoxazol-5-yl-CH₂ |
| 609 | 3-C₆H₅-isoxazol-5-yl-CH₂ |
| 610 | 4-Cl, 3-C₆H₅-isoxazol-5-yl-CH₂ |
| 611 | 4-Br, 3-C₆H₅-isoxazol-5-yl-CH₂ |
| 612 | 4-Br-isoxazol-5-yl-CH₂ |
| 613 | 4-Cl-isoxazol-5-yl-CH₂ |
| 614 | 4-CN-isoxazol-5-yl-CH₂ |
| 615 | 4-CH₃-isoxazol-5-yl-CH₂ |
| 616 | thiazol-2-yl-CH₂ |
| 617 | 4-Br-thiazol-2-yl-CH₂ |
| 618 | 4-Cl-thiazol-2-yl-CH₂ |
| 619 | 4-CN-thiazol-2-yl-CH₂ |
| 620 | 4-CH₃-thiazol-2-yl-CH₂ |
| 621 | 5-Br-thiazol-2-yl-CH₂ |
| 622 | 5-Cl-thiazol-2-yl-CH₂ |
| 623 | 5-CN-thiazol-2-yl-CH₂ |
| 624 | 5-CH₃-thiazol-2-yl-CH₂ |
| 625 | thiazol-4-yl-CH₂ |
| 626 | 2-Br-thiazol-4-yl-CH₂ |
| 627 | 2-Cl-thiazol-4-yl-CH₂ |
| 628 | 2-CN-thiazol-4-yl-CH₂ |
| 629 | 2-CH₃-thiazol-4-yl-CH₂ |
| 630 | 5-Br-thiazol-4-yl-CH₂ |
| 631 | 5-Cl-thiazol-4-yl-CH₂ |
| 632 | 5-CN-thiazol-4-yl-CH₂ |
| 633 | 5-CH₃-thiazol-4-yl-CH₂ |
| 634 | thiazol-5-yl-CH₂ |
| 635 | 4-Br-thiazol-5-yl-CH₂ |
| 636 | 4-Cl-thiazol-5-yl-CH₂ |
| 637 | 4-CN-thiazol-5-yl-CH₂ |
| 638 | 4-CH₃-thiazol-5-yl-CH₂ |
| 639 | 2-Br-thiazol-5-yl-CH₂ |
| 640 | 2-Cl-thiazol-5-yl-CH₂ |
| 641 | 2-CN-thiazol-5-yl-CH₂ |
| 642 | 2-CH₃-thiazol-5-yl-CH₂ |
| 643 | isothiazol-3-yl-CH₂ |
| 644 | 4-Br-isothiazol-3-yl-CH₂ |
| 645 | 4-Cl-isothiazol-3-yl-CH₂ |
| 646 | 4-CN-isothiazol-3-yl-CH₂ |
| 647 | 4-CH₃-isothiazol-3-yl-CH₂ |
| 648 | 5-Br-isothiazol-3-yl-CH₂ |
| 649 | 5-Cl-isothiazol-3-yl-CH₂ |
| 650 | 5-CN-isothiazol-3-yl-CH₂ |
| 651 | 5-CH₃-isothiazol-3-yl-CH₂ |
| 652 | isothiazol-4-yl-CH₂ |
| 653 | 3-Br-isothiazol-4-yl-CH₂ |
| 654 | 3-Cl-isothiazol-4-yl-CH₂ |
| 655 | 3-CN-isothiazol-4-yl-CH₂ |
| 656 | 3-CH₃-isothiazol-4-yl-CH₂ |
| 657 | 5-Br-isothiazol-4-yl-CH₂ |
| 658 | 5-Cl-isothiazol-4-yl-CH₂ |
| 659 | 5-CN-isothiazol-4-yl-CH₂ |
| 660 | 5-CH₃-isothiazol-4-yl-CH₂ |
| 661 | 3,5-(CH₃)₂-isothiazol-4-yl-CH₂ |
| 662 | isothiazol-5-yl-CH₂ |
| 663 | 3-Br-isothiazol-5-yl-CH₂ |
| 664 | 3-Cl-isothiazol-5-yl-CH₂ |
| 665 | 3-CN-isothiazol-5-yl-CH₂ |
| 666 | 3-CH₃-isothiazol-5-yl-CH₂ |
| 667 | 4-Br-isothiazol-5-yl-CH₂ |
| 668 | 4-Cl-isothiazol-5-yl-CH₂ |
| 669 | 4-CN-isothiazol-5-yl-CH₂ |
| 670 | 4-CH₃-isothiazol-5-yl-CH₂ |
| 671 | imidazol-2-yl-CH₂ |
| 672 | 1-Cl-imidazol-2-yl-CH₂ |
| 673 | 1-Br-imidazol-2-yl-CH₂ |
| 674 | 1-CN-imidazol-2-yl-CH₂ |
| 675 | 1-CH₃-imidazol-2-yl-CH₂ |
| 676 | 4-Cl-imidazol-2-yl-CH₂ |
| 677 | 4-Br-imidazol-2-yl-CH₂ |
| 678 | 4-CN-imidazol-2-yl-CH₂ |
| 679 | 4-CH₃-imidazol-2-yl-CH₂ |
| 680 | 1-CH₃, 5-Cl-imidazol-2-yl-CH₂ |
| 681 | 1,4-(CH₃)₂-imidazol-2-yl-CH₂ |
| 682 | 1,5-(CH₃)₂-imidazol-2-yl-CH₂ |
| 683 | imidazol-4-yl-CH₂ |
| 684 | 2-Cl-imidazol-4-yl-CH₂ |
| 685 | 2-Br-imidazol-4-yl-CH₂ |
| 686 | 2-CN-imidazol-4-yl-CH₂ |
| 687 | 1-CH₃-imidazol-4-yl-CH₂ |
| 688 | 2-CH₃-imidazol-4-yl-CH₂ |
| 689 | 5-Cl-imidazol-4-yl-CH₂ |
| 690 | 5-Br-imidazol-4-yl-CH₂ |
| 691 | 5-CN-imidazol-4-yl-CH₂ |
| 692 | 5-CH₃-imidazol-4-yl-CH₂ |
| 693 | 1-CH₃, 5-Cl-imidazol-4-yl-CH₂ |
| 694 | 1,2-(CH₃)₂-imidazol-4-yl-CH₂ |
| 695 | 1,5-(CH₃)₂-imidazol-4-yl-CH₂ |
| 696 | pyrazol-3-yl-CH₂ |
| 697 | 5-Br-pyrazol-3-yl-CH₂ |
| 698 | 5-Cl-pyrazol-3-yl-CH₂ |
| 699 | 5-CN-pyrazol-3-yl-CH₂ |
| 700 | 5-CH₃-pyrazol-3-yl-CH₂ |
| 701 | 1-C₆H₅-pyrazol-3-yl-CH₂ |
| 702 | 4-Br-pyrazol-3-yl-CH₂ |
| 703 | 4-Cl-pyrazol-3-yl-CH₂ |
| 704 | 4-CN-pyrazol-3-yl-CH₂ |
| 705 | 4-CH₃-pyrazol-3-yl-CH₂ |
| 706 | 1-CH₃-pyrazol-3-yl-CH₂ |
| 707 | 1,4-(CH₃)₂-pyrazol-3-yl-CH₂ |
| 708 | 1,5-(CH₃)₂-pyrazol-3-yl-CH₂ |
| 709 | 1-CH₃, 4-Cl-pyrazol-3-yl-CH₂ |
| 710 | 1-CH₃, 5-Cl-pyrazol-3-yl-CH₂ |
| 711 | pyrazol-4-yl-CH₂ |
| 712 | 3-Br-pyrazol-4-yl-CH₂ |
| 713 | 3-Cl-pyrazol-4-yl-CH₂ |
| 714 | 3-CN-pyrazol-4-yl-CH₂ |
| 715 | 3-CH₃-pyrazol-4-yl-CH₂ |
| 716 | 1-CH₃-pyrazol-4-yl-CH₂ |
| 717 | 1,5-(CH₃)₂-pyrazol-4-yl-CH₂ |
| 718 | 1,3-(CH₃)₂-pyrazol-4-yl-CH₂ |
| 719 | 1-CH₃, 3-Cl-pyrazol-4-yl-CH₂ |
| 720 | 1-CH₃, 5-Cl-pyrazol-4-yl-CH₂ |
| 721 | pyrazol-5-yl-CH₂ |
| 722 | 3-Br-pyrazol-5-yl-CH₂ |
| 723 | 3-Cl-pyrazol-5-yl-CH₂ |
| 724 | 3-CN-pyrazol-5-yl-CH₂ |
| 725 | 3-CH₃-pyrazol-5-yl-CH₂ |
| 726 | 1-CH₃-pyrazol-5-yl-CH₂ |
| 727 | 4-Br-pyrazol-5-yl-CH₂ |
| 728 | 4-Cl-pyrazol-5-yl-CH₂ |
| 729 | 4-CN-pyrazol-5-yl-CH₂ |
| 730 | 4-CH₃-pyrazol-5-yl-CH₂ |
| 731 | 1,3-(CH₃)₂-pyrazol-5-yl-CH₂ |
| 732 | 1,4-(CH₃)₂-pyrazol-5-yl-CH₂ |
| 733 | 1-CH₃, 3-Cl-pyrazol-5-yl-CH₂ |
| 734 | 1-CH₃, 4-Cl-pyrazol-5-yl-CH₂ |
| 735 | 1,3,4-oxadiazol-5-yl-CH₂ |
| 736 | 2-CH₃-1,3,4-oxadiazol-5-yl-CH₂ |
| 737 | 2-CF₃-1,3,4-oxadiazol-5-yl-CH₂ |
| 738 | 2-OCH₃-1,3,4-oxadiazol-5-yl-CH₂ |
| 739 | 2-Cl-1,3,4-oxadiazol-5-yl-CH₂ |
| 740 | 2-CH(CH₃)₂-1,3,4-oxadiazol-5-yl-CH₂ |
| 741 | 1,3,4-oxadiazol-2-yl-CH₂ |
| 742 | 5-CH₃-1,3,4-oxadiazol-2-yl-CH₂ |
| 743 | 5-CF₃-1,3,4-oxadiazol-2-yl-CH₂ |
| 744 | 5-OCH₃-1,3,4-oxadiazol-2-yl-CH₂ |
| 745 | 5-Cl-1,3,4-oxadiazol-2-yl-CH₂ |
| 746 | 5-CH(CH₃)₂-1,3,4-oxadiazol-2-yl-CH₂ |
| 747 | 5-C₆H₅-1,3,4-oxadiazol-2-yl-CH₂ |
| 748 | 1,2,4-oxadiazol-3-yl-CH₂ |
| 749 | 5-CH₃-1,2,4-oxadiazol-3-yl-CH₂ |
| 750 | 5-CF₃-1,2,4-oxadiazol-3-yl-CH₂ |
| 751 | 5-OCH₃-1,2,4-oxadiazol-3-yl-CH₂ |
| 752 | 5-Cl-1,2,4-oxadiazol-3-yl-CH₂ |
| 753 | 5-CH(CH₃)₂-1,2,4-oxadiazol-3-yl-CH₂ |
| 754 | 1,2,4-triazol-3-yl-CH₂ |
| 755 | 1-CH₃-1,2,4-triazol-3-yl-CH₂ |
| 756 | 5-CH₃-1,2,4-triazol-3-yl-CH₂ |
| 757 | 5-CF₃-1,2,4-triazol-3-yl-CH₂ |
| 758 | 5-OCH₃-1,2,4-triazol-3-yl-CH₂ |
| 759 | 5-Cl-1,2,4-triazol-3-yl-CH₂ |
| 760 | 5-CH(CH₃)₂-1,2,4-triazol-3-yl-CH₂ |
| 761 | 1-C₆H₅-1,2,4-triazol-3-yl-CH₂ |
| 762 | 1,3,4-thiadiazol-5-yl-CH₂ |
| 763 | 2-CH₃-1,3,4-thiadiazol-5-yl-CH₂ |
| 764 | 2-CF₃-1,3,4-thiadiazol-5-yl-CH₂ |
| 765 | 2-OCH₃-1,3,4-thiadiazol-5-yl-CH₂ |
| 766 | 2-CH₂OCH₃-1,3,4-thiadiazol-5-yl-CH₂ |
| 767 | 2-Cl-1,3,4-thiadiazol-5-yl-CH₂ |
| 768 | 2-CH(CH₃)₂-1,3,4-thiadiazol-5-yl-CH₂ |
| 769 | 1,3,4-thiadiazol-2-yl-CH₂ |
| 770 | 5-CH₃-1,3,4-thiadiazol-2-yl-CH₂ |
| 771 | 5-CF₃-1,3,4-thiadiazol-2-yl-CH₂ |
| 772 | 5-OCH₃-1,3,4-thiadiazol-2-yl-CH₂ |
| 773 | 5-Cl-1,3,4-thiadiazol-2-yl-CH₂ |
| 774 | 5-CH(CH₃)₂-1,3,4-thiadiazol-2-yl-CH₂ |
| 775 | 5-C₆H₅-1,3,4-thiadiazol-2-yl-CH₂ |
| 776 | 1,2,4-thiadiazol-3-yl-CH₂ |
| 777 | 5-CH₃-1,2,4-thiadiazol-3-yl-CH₂ |
| 778 | 5-CF₃-1,2,4-thiadiazol-3-yl-CH₂ |
| 779 | 5-OCH₃-1,2,4-thiadiazol-3-yl-CH₂ |
| 780 | 5-Cl-1,2,4-thiadiazol-3-yl-CH₂ |
| 781 | 5-CH(CH₃)₂-1,2,4-thiadiazol-3-yl-CH₂ |
| 782 | pyrrol-2-yl-CH₂ |
| 783 | 4-Cl-pyrrol-2-yl-CH₂ |
| 784 | 4-Br-pyrrol-2-yl-CH₂ |
| 785 | 4-CH₃-pyrrol-2-yl-CH₂ |
| 786 | 4-C₆H₅-pyrrol-2-yl-CH₂ |
| 787 | benzimidazol-2-yl-CH₂ |
| 788 | chinolin-2-yl-CH₂ |
| 789 | oxiranyl-CH₂ |
| 790 | 2-CH₃-oxiran-2-yl-CH₂ |
| 791 | 2-CH₃-oxiran-3-yl-CH₂ |
| 792 | 2,2-(CH₃)₂-oxiran-3-yl-CH₂ |
| 793 | 2,3-(CH₃)₂-oxiran-3-yl-CH₂ |
| 794 | 2,3,3-(CH₃)₃-oxiran-2-yl-CH₂ |
| 795 | oxiranyl-CH(CH₃) |
| 796 | 2-CH₃-oxiran-2-yl-CH(CH₃) |
| 797 | 2-CH₃-oxiran-3-yl-CH(CH₃) |
| 798 | 2,2-(CH₃)₂-oxiran-3-yl-CH(CH₃) |
| 799 | 2,3-(CH₃)₂-oxiran-3-yl-CH(CH₃) |
| 800 | 2,3,3-(CH₃)₃-oxiran-2-yl-CH(CH₃) |
| 801 | 1,1-Cl₂-cyclopropan-2-yl-CH₂ |
| 802 | 2-CH₃, 1,1-Cl₂-cyclopropan-2-yl-CH₂ |
| 803 | 2-CH₃, 1,1-Cl₂-cyclopropan-3-yl-CH₂ |
| 804 | 2,2-(CH₃)₂, 1,1-Cl₂-cyclopropan-3-yl-CH₂ |
| 805 | 2,3-(CH₃)₂, 1,1-Cl₂-cyclopropan-3-yl-CH₂ |
| 806 | 2,3,3-(CH₃)₃, 1,1-Cl₂-cyclopropan-2-yl-CH₂ |
| 807 | 1,1-Cl₂-cyclopropan-2-yl-CH(CH₃) |
| 808 | 2-CH₃, 1,1-Cl₂-cyclopropan-2-yl-CH(CH₃) |
| 809 | 2-CH₃, 1,1-Cl₂-cyclopropan-3-yl-CH(CH₃) |
| 810 | 2,2-(CH₃)₂, 1,1-Cl₂-cyclopropan-3-yl-CH(CH₃) |
| 811 | 2,3-(CH₃)₂, 1,1-Cl₂-cyclopropan-3-yl-CH(CH₃) |
| 812 | 2,3,3-(CH₃)₃, 1,1-Cl₂-cyclopropan-2-yl-CH(CH₃) |
| 813 | 1,1-Br₂-cyclopropan-2-yl-CH₂ |
| 814 | 2-CH₃, 1,1-Br₂-cyclopropan-2-yl-CH₂ |
| 815 | 2-CH₃, 1,1-Br₂-cyclopropan-3-yl-CH₂ |
| 816 | 2,2-(CH₃)₂, 1,1-Br₂-cyclopropan-3-yl-CH₂ |
| 817 | 2,3-(CH₃)₂, 1,1-Br₂-cyclopropan-3-yl-CH₂ |
| 818 | 2,3,3-(CH₃)₃, 1,1-Br₂-cyclopropan-2-yl-CH₂ |
| 819 | 1,1-Br₂-cyclopropan-2-yl-CH(CH₃) |
| 820 | 2-CH₃, 1,1-Br₂-cyclopropan-2-yl-CH(CH₃) |
| 821 | 2-CH₃, 1,1-Br₂-cyclopropan-3-yl-CH(CH₃) |
| 822 | 2,2-(CH₃)₂, 1,1-Br₂-cyclopropan-3-yl-CH(CH₃) |
| 823 | 2,3-(CH₃)₂, 1,1-Br₂-cyclopropan-3-yl-CH(CH₃) |
| 824 | 2,3,3-(CH₃)₃, 1,1-Br₂-cyclopropan-2-yl-CH(CH₃) |
| 825 | CH₂CH=CH₂ |
| 826 | CH₂CCl=CH₂ |
| 827 | CH₂CH=CHCl (E) |
| 828 | CH₂CH=CHCl (Z) |
| 829 | CH₂CCl=CHCl (E) |
| 830 | CH₂CCl=CHCl (Z) |
| 831 | CH₂CH=CCl₂ |
| 832 | CH₂CCl=CCl₂ |
| 833 | CH₂CBr=CH₂ |
| 834 | CH₂CH=CHBr (E) |
| 835 | CH₂CH=CHBr (Z) |
| 836 | CH₂CBr=CHBr (E) |
| 837 | CH₂CBr=CHBr (Z) |
| 838 | CH₂CH=CBr₂ |
| 839 | CH₂CBr=CBr₂ |
| 840 | CH₂C(CH₃)=CH₂ |
| 841 | CH₂CH=CHCH₃ (E) |
| 842 | CH₂CH=CHCH₃ (Z) |
| 843 | CH₂C(CH₃)=CHCH₃ (E) |
| 844 | CH₂C(CH₃)=CHCH₃ (Z) |
| 845 | CH₂CH=C(CH₃)₂ |
| 846 | CH₂CH₂CH=CH₂ |
| 847 | CH₂CCl=CHCH₃ (E) |
| 848 | CH₂CCl=CHCH₃ (Z) |
| 849 | CH₂CH=CClCH₃ (E) |
| 850 | CH₂CH=CClCH₃ (Z) |
| 851 | CH₂C(CH₃)=C(CH₃)₂ |
| 852 | CH₂CBr=CHCH₃ (E) |
| 853 | CH₂CBr=CHCH₃ (Z) |
| 854 | CH₂CH=CBrCH₃ (E) |
| 855 | CH₂CH=CBrCH₃ (Z) |
| 856 | CH₂CH=CHCH₂Cl (E) |
| 857 | CH₂CH=CHCH₂Cl (Z) |
| 858 | CH₂CH=CHCH₂CH₃ (E) |
| 859 | CH₂CH=CHCH₂CH₃ (Z) |
| 860 | CH₂CH=CHCH₂Br (E) |
| 861 | CH₂CH=CHCH₂Br (Z) |
| 862 | CH₂CCl=CClCH₂Cl (E) |
| 863 | CH₂CCl=CClCH₂Cl (Z) |
| 864 | CH₂CF=CH₂ |
| 865 | CH₂CH=CHF (E) |
| 866 | CH₂CH=CHF (Z) |
| 867 | CH₂CH=CF₂ |
| 868 | CH₂CF=CHF (E) |
| 869 | CH₂CF=CHF (Z) |
| 870 | CH(CH₃)CH=CH₂ |
| 871 | CH(CH₃)CCl=CH₂ |
| 872 | CH(CH₃)CH=CHCl (E) |
| 873 | CH(CH₃)CH=CHCl (Z) |
| 874 | CH(CH₃)CCl=CHCl (E) |
| 875 | CH(CH₃)CCl=CHCl (Z) |
| 876 | CH(CH₃)CH=CCl₂ |
| 877 | CH(CH₃)CCl=CCl₂ |
| 878 | CH(CH₃)CBr=CH₂ |
| 879 | CH(CH₃)CH=CHBr (E) |
| 880 | CH(CH₃)CH=CHBr (Z) |
| 881 | CH(CH₃)CBr=CHBr (E) |
| 882 | CH(CH₃)CBr=CHBr (Z) |
| 883 | CH(CH₃)CH=CBr₂ |
| 884 | CH(CH₃)CBr=CBr₂ |
| 885 | CH(CH₃)C(CH₃)=CH₂ |
| 886 | CH(CH₃)CH=CHCH₃ (E) |
| 887 | CH(CH₃)CH=CHCH₃ (Z) |
| 888 | CH(CH₃)C(CH₃)=CHCH₃ (E) |
| 889 | CH(CH₃)C(CH₃)=CHCH₃ (Z) |
| 890 | CH(CH₃)CH=C(CH₃)₂ |
| 891 | CH(CH₃)CCl=CHCH₃ (E) |
| 892 | CH(CH₃)CCl=CHCH₃ (Z) |
| 893 | CH(CH₃)CH=CClCH₃ (E) |
| 894 | CH(CH₃)CH=CClCH₃ (Z) |
| 895 | CH(CH₃)CBr=CHCH₃ (E) |
| 896 | CH(CH₃)CBr=CHCH₃ (Z) |
| 897 | CH(CH₃)CH=CBrCH₃ (E) |
| 898 | CH(CH₃)CH=CBrCH₃ (Z) |
| 899 | CH(CH₃)CH=CHCH₂Cl (E) |
| 900 | CH(CH₃)CH=CHCH₂Cl (Z) |
| 901 | CH(CH₃)CH=CHCH₂CH₃ (E) |
| 902 | CH(CH₃)CH=CHCH₂CH₃ (Z) |
| 903 | CH(CH₃)CH=CHCH₂Br (E) |
| 904 | CH(CH₃)CH=CHCH₂Br (Z) |
| 905 | CH(CH₃)CCl=CClCH₂Cl (E) |
| 906 | CH(CH₃)CCl=CClCH₂Cl (Z) |
| 907 | CH(CH₃)CF=CH₂ |
| 908 | CH(CH₃)CH=CHF (E) |
| 909 | CH(CH₃)CH=CHF (Z) |
| 910 | CH(CH₃)CH=CF₂ |
| 911 | CH(CH₃)CF=CHF (E) |
| 912 | CH(CH₃)CF=CHF (Z) |
| 913 | CH₂CHClCH=CH₂ |
| 914 | CH₂CH₂CH=C(CH₃)₂ |
| 915 | CH₂CH₂C(CH₃)=CHCH₃ (E) |
| 916 | CH₂CH₂C(CH₃)=CHCH₃ (Z) |
| 917 | CH₂C≡CH |
| 918 | CH₂C≡CCl |
| 919 | CH₂C≡CBr |
| 920 | CH₂C≡CI |
| 921 | CH₂C≡CCH₃ |
| 922 | CH₂C≡CCH₂CH₃ |
| 923 | CH₂C≡CCH₂OH |
| 924 | CH₂C≡CCH₂NH₂ |
| 925 | CH₂C≡CCH₂Cl |
| 926 | CH₂C≡CCH₂OCH₃ |
| 927 | CH₂C≡CCH₂OCH₂CH₃ |
| 928 | CH₂C≡CCH₂SCH₃ |
| 929 | CH₂C≡CCH₂N(CH₃)₂ |
| 930 | CH₂C≡CC₆H₅ |
| 931 | CH₂CH₂C≡CH |
| 932 | CH₂CH₂C≡CCl |
| 933 | CH₂CH₂C≡CBr |
| 934 | CH₂CH₂C≡CI |
| 935 | CH₂CH₂C≡CCH₃ |
| 936 | CH₂CH₂C≡CCH₂CH₃ |
| 937 | CH₂CH₂C≡CCH₂OH |
| 938 | CH₂CH₂C≡CCH₂NH₂ |
| 939 | CH₂CH₂C≡CCH₂Cl |
| 940 | CH₂CH₂C≡CCH₂OCH₃ |
| 941 | CH₂CH₂C≡CCH₂OCH₂CH₃ |
| 942 | CH₂CH₂C≡CCH₂SCH₃ |
| 943 | CH₂CH₂C≡CCH₂N(CH₃)₂ |
| 944 | CH₂CH₂C≡CC₆H₅ |
| 945 | CH₂CH₂CH₂C≡CH |
| 946 | CH₂CH₂CH₂C≡CCl |
| 947 | CH₂CH₂CH₂C≡CBr |
| 948 | CH₂CH₂CH₂C≡CI |
| 949 | CH₂CH₂CH₂C≡CCH₃ |
| 950 | CH₂CH₂CH₂C≡CCH₂CH₃ |
| 951 | CH₂CH₂CH₂C≡CCH₂OH |
| 952 | CH₂CH₂CH₂C≡CCH₂NH₂ |
| 953 | CH₂CH₂CH₂C≡CCH₂Cl |
| 954 | CH₂CH₂CH₂C≡CCH₂OCH₃ |
| 955 | CH₂CH₂CH₂C≡CCH₂OCH₂CH₃ |
| 956 | CH₂CH₂CH₂C≡CCH₂SCH₃ |
| 957 | CH₂CH₂CH₂C≡CCH₂N(CH₃)₂ |
| 958 | CH₂CH₂CH₂C≡CC₆H₅ |
| 959 | CH(CH₃)C≡CH |
| 960 | CH(CH₃)C≡CCl |
| 961 | CH(CH₃)C≡CBr |
| 962 | CH(CH₃)C≡CI |
| 963 | CH(CH₃)C≡CCH₃ |
| 964 | CH(CH₃)C≡CCH₂CH₃ |
| 965 | CH(CH₃)C≡CCH₂OH |
| 966 | CH(CH₃)C≡CCH₂NH₂ |
| 967 | CH(CH₃)C≡CCH₂Cl |
| 968 | CH(CH₃)C≡CCH₂OCH₃ |
| 969 | CH(CH₃)C≡CCH₂OCH₂CH₃ |
| 970 | CH(CH₃)C≡CCH₂SCH₃ |
| 971 | CH(CH₃)C≡CCH₂N(CH₃)₂ |
| 972 | CH(CH₃)C≡CC₆H₅ |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der Verbindungen I gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nicht-ionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate wie Dinitro-(1-methylheptyl)phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)phosphinyl]-3-phenyl-1,2,4-triazol, 2,3-Dicyano-1,4-dithioanthrachinon, 2-Thio-1,3-dithiolo-[4,5-b]-chinoxalin, 1-(Butylcarbamoyl)-2-benzimidazol-carbaminsäuremethylester, 2-Methoxycarbonylamino-benzimidazol, 2-(Furyl-(2))benzimidazol, 2-(Thiazolyl-(4))benzimidazol, N-(1,1,2,2-Tetrachlorethylthio)tetrahydrophthalimid, N-Trichlormethylthio-tetrahydrophthalimid, N-Trichlor-methylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlor-ethylacetal, Piperazin-1,4-diyl-bis-(1-(2,2,2-trichlor-ethyl)formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlor-phenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)benzol, l,2-Bis-3-methoxycarbonyl-2-thioureido)benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol, sowie
verschiedene Fungizide wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl(2)alaninat, DL-N-(2,6-Dimethyl-phenyl) -N-(2'-methoxyacetyl)alanin-methylester, N-(2,6-Dimethylphenyl)-N-chloracetyl-D,L-2-aminobutyrolacton, DL-N-(2,6-Dimethylphenyl)-N-(phenylacetyl)alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorhenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)pentyl]-1H-1,2,4-triazol, 2,4-Difluor-a-(1H-1,2,4-triazolyl-1-methyl)benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)methylsilyl)methyl)-1H-1,2,4-triazol.

Strobilurine wie Methyl-E-methoximino-[a-(o-tolyloxy)-o-tolyl]acetat, Methyl-E-2-{2-[6-(2-cyanophenoxy)pyrimidin-4-yl-oxy]-phenyl}-3-methoxyacrylat, Methyl-E-methoximino-[a-(2-phenoxyphenyl)]acetamid, Methyl-E-methoximino-[a-(2,5-dimethylphenoxy)-o-tolyl]acetamid.

Anilinopyrimidine wie N-(4,6-Dimethylpyrimidin-2-yl)anilin, N-[4-Methyl-6-(1-propinyl)pyrimidin-2-yl]anilin, N-(4-Methyl-6-cyclopropyl-pyrimidin-2-yl)anilin.

Phenylpyrrole wie 4-(2,2-difluor-1,3-benzodioxol-4-yl)pyrrol-3-carbonitril.

Zimtsäureamide wie 3-(4-Chlorphenyl)-3-(3,4-dimethoxyphenyl)acrylsäuremorpholid.

(2RS,3SR)-1-[3-(2-Chlorphenyl)-2-[4-fluorphenyl]oxiran-2-ylmethyl]-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nord-mannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
- I.: 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
- II.: 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
- III.: 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
- IV.: 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
- V.: 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
- VI.: Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
- VII.: 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
- VIII.: 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1

### Darstellung von E-2-Methoxyimino-2-[(2'-phthalimidooxymethyl)-phenyl]essigsäuremethylester

Zu einer Lösung von 150 g (0,52 mol) E-2-Methoxyimino-2-[(2'-brommethyl)-phenyl]essigsäuremethylester und 85 g (0,52 mol) N-Hydroxyphthalimid in 350 ml N-Methylpyrrolidon tropft man 59 g (0,58 mol) Triethylamin und rührt 2 h bei 70°C. Man gießt das Reaktionsgemisch auf 2 l Eiswasser, saugt die ausgefallenen Kristalle ab und nimmt sie in Methylenchlorid auf. Nach Waschen der organischen Phase mit Wasser, Trocknen über Natriumsulfat und Abrotieren des Lösungsmittels erhält man 168 g (88 % Ausbeute) E-2-Methoxyimino-2-[(2'-phthalimidooxymethyl)-phenyl]essigsäuremethylester als hellgraues Pulver vom Schmelzpunkt 152-153°C.
¹H-NMR (CDCl₃): δ = 3,81 (s,3H); 3,95 (s,3H); 5,05(s,2H); 7,12-7,81(m,8H) ppm.

### Beispiel 2

### Darstellung von (E,E)-2-Methoxyimino-2-[2'-[(1"-methyl, 1"-hydroxycarbonyl)-iminooxymethyl]phenyl]-essigsäuremethylester

Zu einer Lösung von 100 g (0,27 mol) der Verbindung aus Beispiel 1 in 1000 ml Methanol gibt man 14 g (0,28 mol) Hydrazinhydrat und läßt 1 h bei Raumtemperatur nachrühren. Anschließend gibt man 30 g Molsieb (3 Ä) und 24 g (0,27 mol) Brenztraubensäure zu und läßt erneut 1 h nachrühren. Das Reaktionsgemisch wird filtriert und das Filtrat eingeengt. Der Rückstand wird anschließen in gesättigter NaHCO₃-Lösung aufgenommen und mit Methylenchlorid extrahiert. Nach Ansäuren der wäßrigen Phase mit 2 M Salzsäure wird erneut mit Methylenchlorid extrahiert, die organische Phase mit Wasser gewaschen, über Natriumsulfat getrocknet und eingeengt. Man erhält so 71 g (86 % Ausbeute) der Titelverbindung.
¹H-NMR (CDCl₃): δ = 2,20 (s,3H); 3,87 (s,3H); 4,04 (s,3H); 5,13 (s,2H); 7,15-7,46 (m,4H); 8,80 (s,br,1H) ppm.

### Beispiel 3

### Darstellung von (E,E)-2-Methoxyimino-2-[2'-[(1"-methyl, 1"-methoxyaminocarbonyl)-iminooxymethyl]-phenyl]essigsäuremethylester

Zu einer Lösung von 71 g (0,23 mol) der Verbindung aus Beispiel 2 in 1000 ml Tetrahydrofuran werden 37 g (0,23 mol) Carbonyldiimidazol gegeben. Nach Beendigung der Gasentwicklung versetzt man portionsweise mit 38 g (0,46 mol) Methoxyaminhydrochlorid und läßt 16 h bei Raumtemperatur nachrühren. Anschließend wird eingeengt, der Rückstand in Methylenchlorid aufgenommen und die organische Phase zunächst mit gesättigter NaHCO₃-Lösung extrahiert. Nach Waschen der organischen Phase mit 2 M Salzsäure und Wasser wird über Natriumsulfat getrocknet und eingeengt. Man erhält so 72,5 g (94 % Ausbeute) der Titelverbindung als weißes Pulver vom Schmelzpunkt 105-108°C.
¹H-NMR (CDCl₃): δ = 2,00 (s,3H); 3,77 (s,3H); 3,88 (s,3H); 4,03 (s,3H); 5,00 (s,2H); 7,13-7,42 (m,4H); 9,19 (s,1H) ppm.

### Beispiel 4*

### Darstellung von (Z,E,E)-2-Methoxyimino-2-[2'-[[1"-methyl,1"-(1"'-ethoxy,1"'-methoxyiminomethyl)]-iminooxymethyl]-phenyl]essigsäuremethylester

Zu einer Mischung aus 10 g (30 mmol) der Verbindung aus Beispiel 3 und 8,2 g (60 mmol) Kaliumcarbonat in 500 ml Dimethylformamid tropft man 6,6 g (60 mmol) Ethylbromid und läßt 60 h bei Raumtemperatur rühren. Man gießt das Reaktionsgemisch auf Wasser und extrahiert mit Methyl-tert.-butylether. Die vereinigten organischen Phasen werden mit Wasser gewaschen, über Natriumsulfat getrocknet und eingeengt. Nach Säulenchromatographie an Kieselgel (Methyl-tert.-butylether/Hexan) erhält man 6,0 g (55 % Ausbeute) der Titelverbindung.
¹H-NMR (CDCl₃): δ = 1,22 (t,3H); 1,99 (s,3H); 3,86 (s,3H); 3,88 (s,3H); 4,04 (s,3H); 4,11 (q,2H); 5,10 (s,2H); 7,16-7,48 (m,4H) ppm.

### Beispiel 5*

### Darstellung von (Z,E,E)-2-Methoxyimino-2-[2'-[[1"-methyl,1"-(1"'-ethoxy,1"'-methoxyiminomethyl)]-iminooxymethyl]phenyl]-essigsäuremethylamid

6,0 g (16 mmol) der Verbindung aus Beispiel 4 werden in 100 ml Tetrahydrofuran gelöst, mit 20 ml 40%iger wäßriger Monomethylaminlösung versetzt und 16 h bei Raumtemperatur gerührt. Anschließend wird mit Wasser versetzt, mit Methyl-tert.-butylether extrahiert und die organische Phase mit Wasser gewaschen, über Natriumsulfat getrocknet und einrotiert. Man erhält so 5,7 g (95 % Ausbeute) der Titelverbindung.
¹H-NMR (CDCl₃): δ = 1,21 (t,3H); 1,97 (s,3H); 2,90 (d,3H); 3,87 (s,3H); 3,94 (s,3H); 4,09 (q,2H); 5,10 (s,2H); 6,77 (s,br,1H); 7,17-7,45 (m,4H) ppm.

### Beispiel 6

### Darstellung von 2,2-Diethoxypropansäure-N-methoxy-amid

Zu einer Mischung aus 250 g (2,84 mol) Brenztraubensäure und 1,15 l Triethylorthoformiat wurde unter Eiskühlung 1 ml konz. H₂SO₄ zugetropft und 1 h bei 5-10°C nachgerührt.

Anschließend gab man portionsweise insgesamt 475 g (2,84 mol) Carbonyldiimidazol zu. Nach Beendigung der Gasentwicklung versetzte man mit 365 g (4,26 mol) Methoxyaminhydrochlorid und ließ 3 h bei Raumtemperatur nachrühren. Der Niederschlag wurde abgetrennt, das Filtrat eingeengt und überschüssiges Triethylorthoformiat Ölpumpenvakuum abgezogen. Der Rückstand wurde in Wasser aufgenommen und mit Methylenchlorid extrahiert, die organische Phase gewaschen, getrocknet und eingeengt. Man erhielt so 568 g der Titelverbindung als braunes Öl, das ohne weitere Reinigung weiter umgesetzt wurde.
¹H - NMR (CDCl₃) : δ = 1,22 (t,6H); 1,52 (s,3H); 3,43 - 3,59 (m,4H); 3,80 (s,3H); 9,28 (s,br,1H) ppm.

### Beispiel 7

### Darstellung von (Z)-N-Methoxy-2,2-diethoxypropanimidsäure-isopropylester

Zu einer Mischung aus 568 g der Verbindung aus Beispiel 6 und 588 g (4,26 mol) Kaliumcarbonat in 1 l Dimethylformamid tropfte man 700 g (5,69 mol) 2-Brompropan und ließ 12 h bei 70°C rühren. Nach Abtrennen des Niederschlages wurde das Filtrat auf Wasser gegossen, mit Methyl-tert.-butylether extrahiert, die organische Phase gewaschen, getrocknet und eingeengt. Man erhielt so 383 g der Titelverbindung als braunes Öl, das lt. ¹H-NMR eine Reinheit von etwa 80 % aufweist.
¹H - NMR (CDCl₃) : δ = 1,20 (t,6H); 1,27 (d,6H); 1,51 (S,3H); 3,47 - 3,64 (m,4H); 3,80 (s,3H); 5,18 (sep,1H) ppm.

### Beispiel 8

### Darstellung von (E)-2-Hydroxyimino, (Z)-N-methoxy-propanimidsäure-isopropylester

Zu einer Lösung von 383 g der Verbindung aus Beispiel 7 in einer Mischung aus 1 l Methanol, 300 ml Wasser und 134 g (1,70 mol) Pyridin, gab man portionsweise 137 g (1,97 mol) Methoxyaminohydrochlorid zu und ließ zunächst 16 h bei Raumtemperatur und anschließend 1 h bei 60°C nachrühren. Die Reaktionsmischung wurde auf verdünnte Salzsäure gegossen und mit Methyl-tert.-butylether extrahiert. Die organische Phase wurde anschließend mit 10%-iger Natronlauge extrahiert und die wäßrige Phase mit verdünnter Salzsäure angesäuert und mit Methyl-tert.-butylether extrahiert. Die Etherphase wurde gewaschen, getrocknet und eingeengt. Nach Säulenchromatographie an Kieselgel (Methyl-tert.-butylether/ n-Hexan) erhielt man 96 g der Titelverbindung als hellgelbes Öl.
¹H - NMR (CDCl₃) : δ = 1,27 (d,6H); 2,06 (s,3H); 3,88 (S,3H); 4,96 (sep,1H); 10,36 (s,br,1H) ppm.

### Beispiel 9

### Darstellung von (Z,E,E)-3-Methoxy-2-[2'-[[1"-methyl, 1"-(1"'-isopropoxy, 1"'-methoxyiminomethyl)]iminooxymethyl]phenyl]acrylsäuremethylester (Verb. I.71)

Zu 0,45 g (14,5 mmol) Natriumhydrid (80 %) in 50 ml Dimethylformamid gab man portionsweise 2,3 g (13,2 mmol) der Verbindung aus Beispiel 8, gelöst in 50 ml Dimethylformamid, und ließ 30 Minuten nachrühren. Anschließend wurde eine Lösung von 3,75 g (13,2 mmol (E)-3-Methoxy-2-[(2'-brommethyl)-phenyl]acrylsäuremethylester in 50 ml Dimethylformamid zugetropft und 16 h bei Raumtemperatur gerührt. Das Reaktionsgemisch wurde auf Wasser gegossen und mit Methyl-tert.-butylether extrahiert. Die organische Phase wurde gewaschen, getrocknet und eingeengt. Nach Säulenchromatographie an Kieselgel (Methyl-tert.-butylether/n-Hexan) erhielt man 1,4 g der Titelverbindung als farblosen Öl.
¹H-NMR (CDCl₃) : δ = 1,22 (d,6H; 2,01 (s,3H); 3,67 (s,3H); 3,81 (s,3H); 3,87 (s,3H); 4,63 (sep,1H); 5,13(s,2H); 7,11 - 7,47 (m,4H); 7,57 (s,1H) ppm.

### Beispiel 10

### Darstellung von (Z,E,E)-2-Methoxyimino-2-[2'-[[1"-methyl, 1"-(1"'-Methoxyiminomethyl)]iminooxymethyl]phenyl]essigsäuremethylester

Eine Mischung aus 50 g (0,15 mol) der Verbindung aus Beispiel 3 und 117 g (0,45 mol) Triphenylphosphin in 1000 ml Acetonitril wurde portionsweise mit 148 g (0,45 mol) Tetrabrommethan versetzt. Die so erhaltene Mischung wurde 100 h refluxiert, anschließend ließ man auf Raumtemperatur (≈25°C) abkühlen. Die Reaktionsmischung wurde von Feststoffen befreit. Vom Filtrat wurde unter vermindertem Druck das Lösungsmittel abdestilliert und der so erhaltene Rückstand wurde säulenchromatographisch [Kieselgel; tert.-Butyl-methylether/Hexan] gereinigt. Zur weiteren Reinigung wurde das Produkt umkristalliert [Methylenchlorid/Hexan]. Man erhielt 34 g (58 %) der Titelverbindung als farbloses Pulver [Fp.: 103-105°C].
¹H-NMR (δ in ppm; CDCl₃) : 2,10 (s,3H); 3,86 (s,3H); 4,05 (s,3H); 4,10 (s,3H); 5,16 (s,2H); 7,16 - 7,51 (m,4H) ppm.

### Beispiel 11

### Darstellung von (Z,E,E)-2-Methoxyimino-2-[2'-[[1"-methyl, 1"-(1"'-brom, 1"'-Methoxyiminomethyl)]iminooxymethyl]-phenyl]essigsäure-N-methylamid

Eine Lösung von 8 g (20 mmol) der Verbindung aus Beispiel 10 in 200 ml Tetrahydrofuran wurde mit 50 ml Methylamin-Lösung (40 %-ig in Wasser) versetzt. Nach 20 min bei Raumtemperatur (ca.25°C) wurde das Reaktionsgemisch in gekühlte 2n Salzsäure gegossen. Das Produkt wurde aus der so erhaltenen Mischung mit tert.-Butylmethylether extrahiert. Aus der Etherphase erhielt man nach üblicher Aufarbeitung 7,7 g (96 %) der Titelverbindung als farbloses Pulver [Fp.; 135-137°C].
¹H-NMR (δ in ppm; CDCl₃) : 2,09 (s,3H); 2,88 (d,3H); 3,94 (s,3H); 4,08 (s,3H); 5,15 (s,2H); 6,74 (s,br,1H); 7,15 - 7,51 (m,4H) ppm.

### Beispiel 12

### Darstellung von (Z,E,E)-2-Methoxyimino-2-[2'-[[1"-methyl, 1"-(1"'-tert.-buthylthio, 1"'-methoxyiminomethyl)]iminooxymethyl]phenyl]essigsäure-N-methylamid (Verb. I 54)

Zu einer Lösung von 1,5 g (3,8 mmol) der Verbindung aus Beispiel 11 in 50 ml Dimethylformamid gab man 0,47 g (4,2 mmol) Natrium-2-methyl-2-propanthiolat und ließ 16 h bei Raumtemperatur nachrühren. Nach erneuter Zugabe von 0,47 g (4,2 mmol) Natrium-2-propanthiolat und 16-stündigem Rühren wurde das Reaktionsgemisch auf Wasser gogossen mit Methyl-tert.-butylether extrahiert und in üblicher Weise aufgearbeitet. Man erhielt so 1,4 g der Titelverbindung als gelbes Öl.
¹H-NMR (CDCl₃) : δ = 1,30 (s,9H); 2,07 (s,3H); 2,88 (d,3H); 3,93 (s,3H); 4,02 (s,3H); 5,11 (s,2H); 6,74 (s,br,1H); 7,13 -7,47 (m,4H) ppm.

### Beispiel 13

### Darstellung von (E,E)-3-Methyl-2-[2'-[[1"-methyl, 1"-methoxycarbonyl]iminooxymethyl]phenyl]-acrylsäuremethylester

Zu 4,6 g (0,155 mol) Natriumhydrid (80 %) in 80 ml Dimethylformamid gab man portionsweise 16,5 g (0,141 mol) (E)-2-Hydroxyiminopropionsäuremethylester, gelöst in 50 ml Dimethylformamid, wobei sich das Reaktionsgemisch bis auf 40°C erwärmte. nach Abkühlen auf Raumtemperatur ließ man 30 Minuten nachrühren, tropfte anschließend eine Lösung von 37,9 g (0,141 mol) (E)-2-Methyl-2-[(2'-brommethyl)phenyl]acrylsäuremethylester in 80 ml Dimethylformamid zu und ließ 16 h bei Raumtemperatur rühren. Das Reaktionsgemisch wurde auf Wasser gegossen und mit Methyl-tert.-butylether extrahiert und in üblicher Weise aufgearbeitet. Nach Säulenchromatographie an Kieselgel (Methyl-tert.-butylether/n-Hexan) erhielt man 34,6 g der Titelverbindung als farblosen Öl.
¹H-NMR (CDCl₃) : δ = 1,61 (d,3H); 2,03 (s,3H); 3,68 (s,3H); 3,84 (s,3H); 5,08 - 5,20 (m,2H); 7,02 - 7,50 (m,5H) ppm.

### Beispiel 14

### Darstellung von (E,E)-3-Methyl-2-[2'-[[1"-methyl, 1"-hydroxycarbonyl]iminooxymethyl]phenyl]-acrylsäuremethylester

Eine Lösung von 5,6 g (19,2 mmol) der Verbindung aus Beispiel 13 in 6 ml Methanol wurde mit 21 ml einer 1 M Lithiumhydroxidlösung versetzt und 16 Stunden bei Raumtemperatur gerührt. Das Reaktionsgemisch wurde mit Methyl-tert.-butylether extrahiert, die wäßrige Phase mit verdünnter Salzsäure angesäuert und anschließend mit Methyl-tert.-butylether extrahiert. Die organische Phase wurde getrocknet und eingeengt. Man erhielt so 5,3 g der Titelverbindung als gelbes Öl.
¹H-NMR (CDCl₃) : δ = 1,61 (d,3H); 2,02 (s,3H); 3,72 (s,3H); 5,13 (s,2H); 7,05 - 7,48 (m,5H) ppm.

### Beispiel 15

### Darstellung von (E,E)-3-Methyl-2-[2'-[[1"-methyl, 1"-methoxyaminocarbonyl]iminooxymethyl]phenyl]-acrylsäuremethylester

Zu einer Lösung von 5,3 g (18,2 mmol) der Verbindung aus Beispiel 14 in 80 ml Tetrahydrofuran wurden 3,1 g (18,2 mmol) Carbonyldiimidazol gegeben. Nach Beendigung der Gasentwicklung versetzte man portionsweise mit 3,2 g (36 mmol) Methoxyaminhydrochlorid und ließ 60 h bei Raumtemperatur nachrühren. Der Niederschlag wurde abgetrennt, das Filtrat auf Wasser gegossen und mit Methyl-tert.-butylether extrahiert. Nach üblicher Aufarbeitung und Säulenchromatographie an Kieselgel (Methyl-tert.-butylether/n-Hexan) erhielt man 4,0 g der Titelverbindung als farbloses Öl.
¹H-NMR (CDCl₃) : δ = 1,61 (d,3H); 1,99 (s,3H); 3,73 (s,3H); 3,79 (s,3H); 5,02 (s,2H); 7,05 - 7,46 (m,5H); 9,23 (s,br,1H) ppm.

### Beispiel 16

### Darstellung von (Z,E,E)-3-Methyl-2-[2'-[[1"-methyl, 1"-allyloxy, 1"'-methoxyiminomethyl)]-iminooxymethyl]phenyl]acrylsäuremethylester (Verb. I. 68)

Zu einer Mischung aus 2,0 g (6,3 mmol) der Verbindung aus Beispiel 15 und 3,7 g (27 mmol) Kaliumcarbonat in 50 ml Dimethylformamid gab man 3,3 g (27 mol) Allylbromid und ließ 16 h bei Raumtemperatur rühren. Man goß das Reaktionsgemisch auf Wasser und extrahierte mit Methyl-tert.-butylether. nach üblicher Aufarbeitung und Säulenchromatographie an Kieselgel(Methyl-tert.-butylether/n-Hexan) erhielt man 1,1 g der Titelverbindung als gelbes Öl.
¹H-NMR (CDCl₃) : δ = 1,61 (d,3H); 1,98 (s,3H); 3,68 (s,3H); 3,87 (s,3H); 4,60 (d,2H); 5,03 - 5,09 (m,2H); 5,15 - 5,26 (m,2H); 5,82 - 5,97 (m,1H); 7,03 - 7,48 (m,5H) ppm.

* Die Beispiele sind nicht erfindungsgemäß

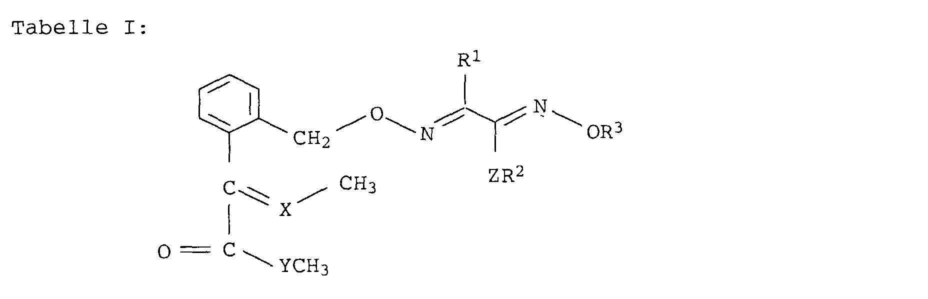

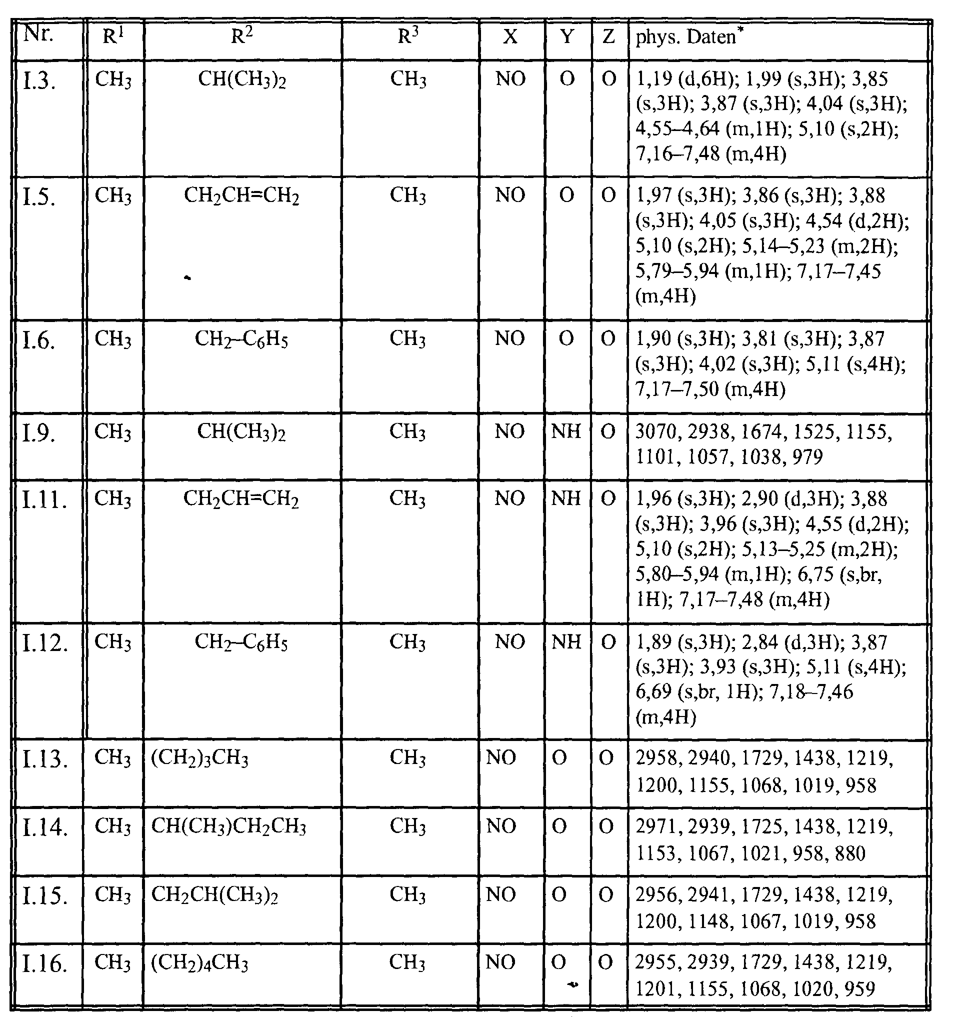

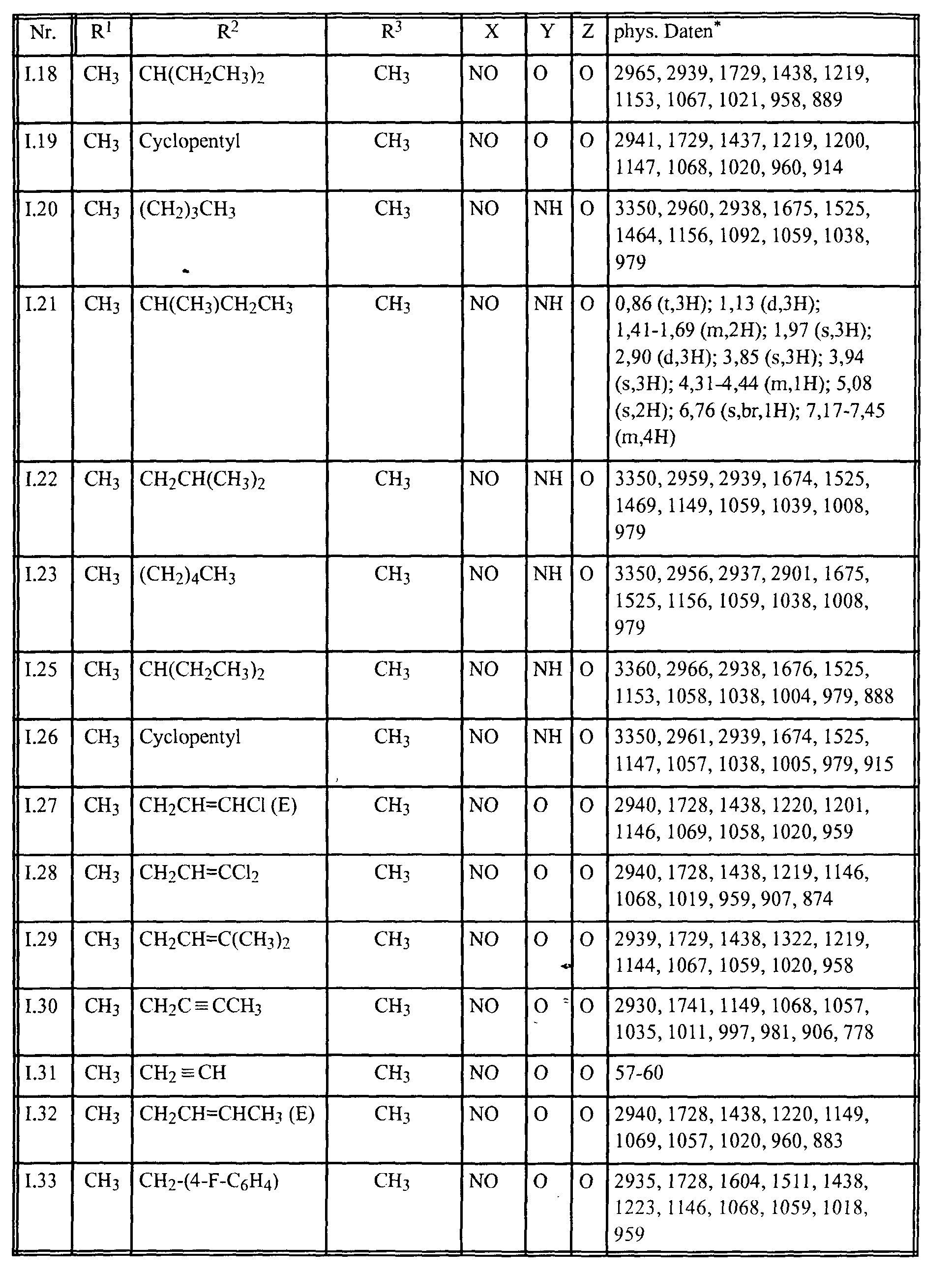

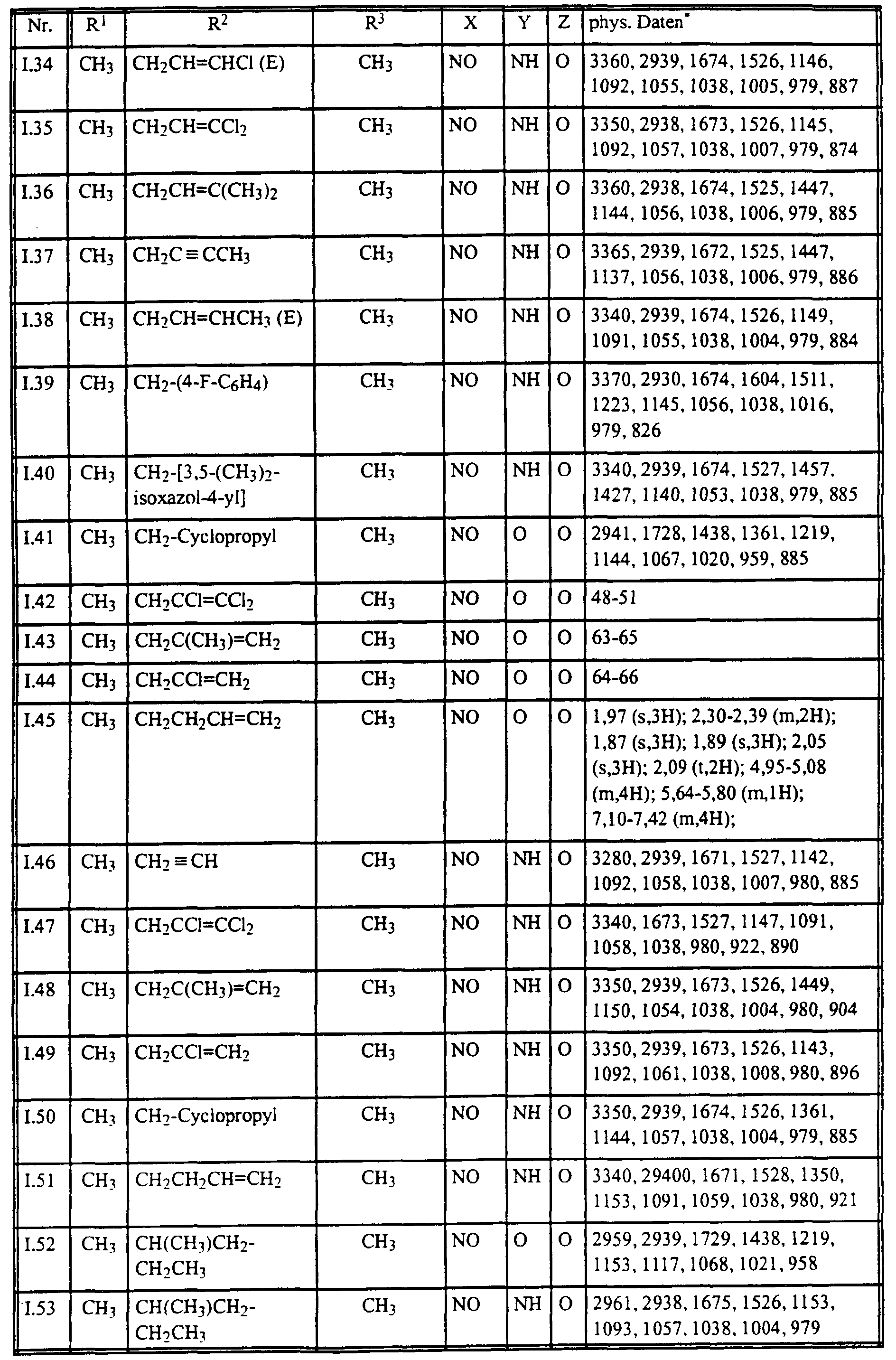

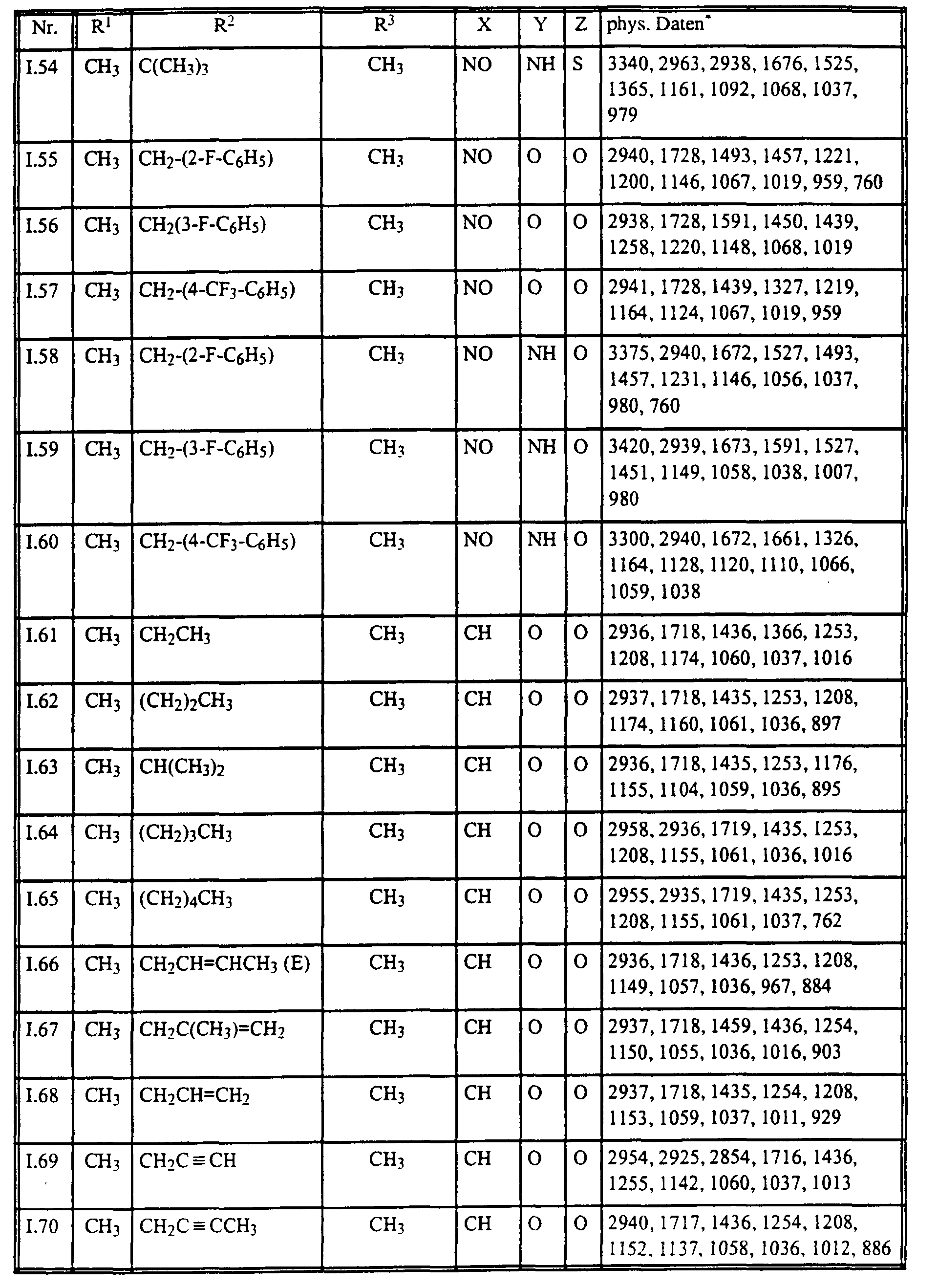

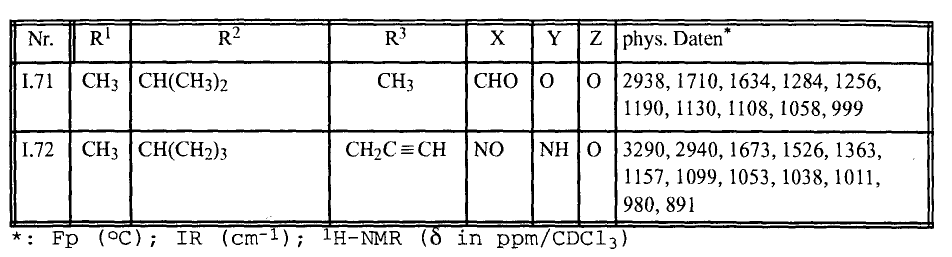

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Als Vergleichsverbindungen dienten die bekannten Wirkstoffe **A** (Beispiel Nr. 657, Tabelle 5 der WO-A 95/21154), **B** (Beispiel Nr. 657, Tabelle 7 der WO-A 95/21153) und **C** (Beispiel Nr. 662, Tabelle 5 der WO-A 95/21154).

Wirkung gegen Plasmopara viticola *(Rebenperonospora)* Topfreben (Sorte: "Müller Thurgau") wurden mit der Wirkstoffaufbereitung tropfnaß gespritzt (Aufwandmenge: 16 ppm). Nach 8 Tagen wurden die Pflanzen mit einer Zoosporenaufschwemmung des Pilzes *Plasmopara viticola* besprüht und 5 Tage bei 20-30 °C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Versuch zeigten die mit den erfindungsgemäßen Verbindungen I.03, I.06, I.08, I.09, I.12, I.13, I.14, I.16, I.19, I.20, I.21, I.22, I.23, I.25, I.26, I.27, I.28, I.29, I.30, I.31, I.33, I.34, I.35, I.37, I.38, I.39, I.40, I.42, I.43, I.44, I.45, I.46, I.47, I.49, I.51, I.53, I.54,I.55, I.56, I.57, I.58, I.59, I.61, I.62, I.63, I.64, I.65, I.66, I.69, I.70, I.71 und I.72 behandelten Pflanzen einen Befall von 25% und weniger, während die mit den bekannten Wirkstoffen **A**, **B** und **C** behandelten Pflanzen zu 60% befallen waren. Die unbehandelten (Kontroll-) Pflanzen waren zu 70% befallen.

### Wirkung gegen Pyricularia oryzae (Reisbrand)

Reiskeimlinge (Sorte: "Tai Nong 67") wurden mit der Wirkstoffaufbereitung tropfnaß gespritzt (Aufwandmenge: 16 ppm). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99 % bewahrt. Die Beurteilung erfolgte visuell.

In diesem Versuch zeigten die mit den erfindungsgemäßen Verbindungen I.03, I.06, I.09, I.12, I.13, I.14, I.15, I.16, I.18, I.19, I.20, I.21, I.22, I.23, I.25, I.26, I.47, I.48, I.49, I.50, I.52, I.53, I.54, I.56, I.57, I.58, I.59, I.61, I.62, I.63, I.64, I.65, I.66, I.67, I.68, I.69, I.70 und I.71 behandelten Pflanzen einen Befall von 15% und weniger, während die mit den bekannten Wirkstoffen **A**, **B** und **C** behandelten Pflanzen zu 40% (im Fall von **B**) bzw. 60% befallen waren. Die unbehandelten (Kontroll-) Pflanzen waren zu 80% befallen.

### Beispiele zur Wirkung gegen tierische Schädlinge

Die Wirkung der Verbindungen der allgemeinen Formel I gegen tierische Schädlinge ließ sich durch folgende Versuche zeigen

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Hydroximsäurederivate der Formel I 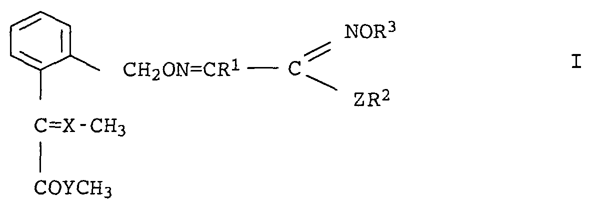 in der die Substituenten die folgende Bedeutung haben:
X CH, CHO oder NO;
Y O oder NH;
Z O oder S;
R¹ Wasserstoff, C₁-C₆-Alkyl oder C₁-C₄-Halogenalkyl;
R² C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
- Cyano, Nitro, Amino, Hydroxy,
- C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl, C₁-C₄-Alkyl, C₁-C₄-Halogenalkyl, C₁-C₄-Alkylcarbonyl, C₁-C₆-Alkoxy, C₁-C₆-Halogenalkoxy, C₁-C₆-Alkoxycarbonyl, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino, C₁-C₆-Alkylaminocarbonyl, Di-C₁-C₆-alkylaminocarbonyl, C₂-C₆-Alkenyloxy und Phenyl;
C₃-C₆-Cycloalkyl, wobei diese Gruppen partiell oder vollständig halogeniert sein können und/oder einen bis drei der folgenden Reste tragen können:
- Cyano, Nitro, Amino, Hydroxy,
- C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino;
R³ C₁-C₆-Alkyl, C₂-C₆-Alkenyl oder C₂-C₆-Alkinyl, wobei diese Gruppen partiell oder vollständig halogeniert sein können und/oder einen bis drei der folgenden Reste tragen können:
- Cyano, C₁-C₃-Alkoxy,
- Oxiranyl oder C₃-C₆-Cycloalkyl, wobei die cyclischen Reste ihrerseits partiell oder vollständig halogeniert sein können und/oder eine bis drei C₁-C₄-Alkylgruppen tragen können;
ausgenommen Hydroximsäurederivate der Formel I, in der
a)
X NO und
Y O oder NH bedeuten; oder
b)
X CHO und
Y O bedeuten;
R¹,R³ Methyl; und
R² Ethyl, 2,2,2-Trifluorethyl, 2-Chlorethyl, n-Propyl, i-Pentyl, tert.-Pentyl oder n-Hexyl bedeuten.

2. Verbindungen der Formel I gemäß Anspruch 1, in denen R² die folgenden Bedeutung hat:
C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
- C₃-C₆-Cycloalkyl, Phenyl oder Oxiranyl, wobei die cyclischen Reste ihrerseits partiell oder vollständig halogeniert sein können und/oder eine bis drei der folgenden Gruppen tragen können: Cyano, C₁-C₄-Alkyl, C₁-C₄-Halogenalkyl, C₁-C₆-Alkoxy, C₁-C₆-Halogenalkoxy, C₁-C₆-Alkoxycarbonyl, C₁-C₆-Alkylthio, C₁-C₆-Halogenalkylthio und Phenyl;
C₃-C₆-Cycloalkyl, wobei diese Gruppen partiell oder vollständig halogeniert sein können und/oder einen bis drei der folgenden Reste tragen können:
- Cyano, C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio.

3. Verfahren zur Herstellung der Verbindungen der Formel I gemäß Anspruch 1, dadurch gekennzeichnet, daß man ein Benzylderivat der Formel II 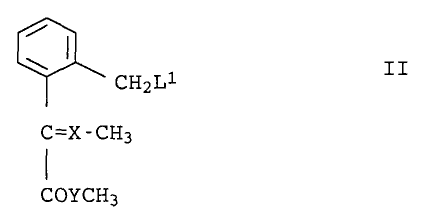 in der L¹ für eine nucleophil austauschbare Abgangsgruppe steht, in an sich bekannter Weise mit einem Hydroxyimin der Formel III 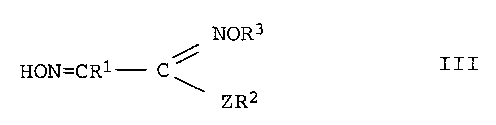 umsetzt.

4. Verfahren zur Herstellung der Verbindungen der Formel I gemäß Anspruch 1, dadurch gekennzeichnet, daß man ein Benzylderivat der Formel II gemäß Anspruch 4 in an sich bekannter Weise mit einem Dihydroxyimin der Formel IV 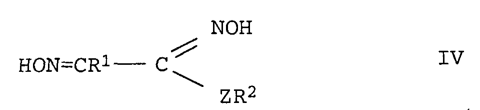 zu einer Verbindung der Formel V 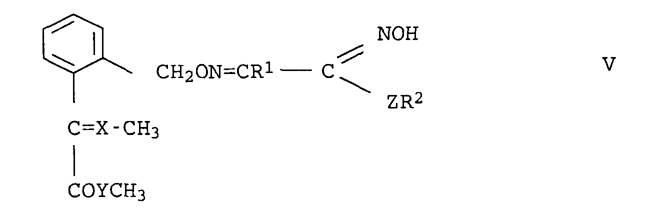 umsetzt und V anschließend mit einer Verbindung der Formel VIa
R³-L² VIa
in der L² für eine nucleophil austauschbare Abgangsgruppe steht, zu I umsetzt.

5. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

6. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

7. Verfahren zur Bekämpfung von tierischen Schädlingen, dadurch gekennzeichnet, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

8. Verbindungen der Formel III 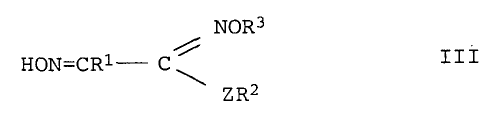 in der die Substituenten Z und R¹ bis R³ die in Anspruch 1 gegebene Bedeutung haben.

9. Verwendung der Verbindungen III gemäß Anspruch 3 als Zwischenprodukte.

10. Verbindungen der Formel Xa 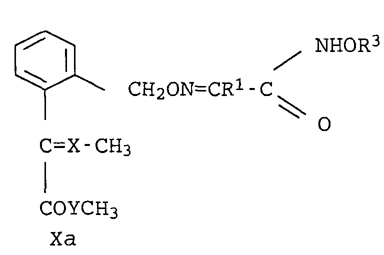 in der die Substituenten X, Y, R¹ und R³ die in Anspruch 1 gegebene Bedeutung haben.

11. Verwendung der Verbindungen Xa gemäß Anspruch 10 als Zwischenprodukte.

## Claims

1. A hydroximic acid derivative of the formula I 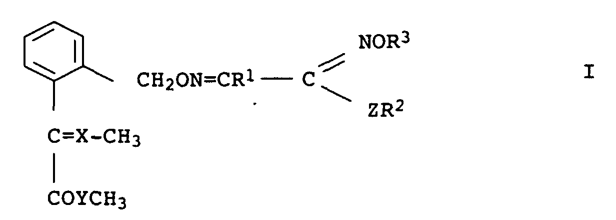 where the substituents have the following meanings:
X is CH, CHO or NO;
Y is O or NH;
Z is O or S;
R¹ is hydrogen, C₁-C₆-alkyl or C₁-C₄-haloalkyl;
R² is C₂-C₆-alkyl, C₃-C₆-alkenyl or C₃-C₆-alkynyl, it being possible for these groups to be partially or fully halogenated and/or to have attached to them one to three of the following radicals:
- cyano, nitro, amino, hydroxyl,
- C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino,
- C₃-C₆-cycloalkyl, aryl, hetaryl or oxiranyl, it being possible for the cyclic radicals, in turn, to be partially or fully halogenated and/or to have attached to them one to three of the following groups: cyano, nitro, amino, aminocarbonyl, aminothiocarbonyl, C₁-C₄-alkyl, C₁-C₄-haloalkyl, C₁-C₄-alkylcarbonyl, C₁-C₆-alkoxy, C₁-C₆-haloalkoxy, C₁-C₆-alkoxycarbonyl, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino, C₁-C₆-alkylaminocarbonyl, di-C₁-C₆-alkylaminocarbonyl, C₂-C₆-alkenyloxy and phenyl;
C₃-C₆-cycloalkyl, it being possible for these groups to be partially or fully halogenated and/or to have attached to them one to three of the following radicals:
- cyano, nitro, amino, hydroxyl,
- C₁-C₄-alkyl, C₁-C₄-haloalkyl, C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino;
R³ is C₁-C₆-alkyl, C₂-C₆-alkenyl or C₂-C₆-alkynyl, it being possible for these groups to be partially or fully halogenated and/or to have attached to them one to three of the following radicals:
- cyano, C₁-C₃-alkoxy,
- oxiranyl or C₃-C₆-cycloalkyl, it being possible for the cyclic radicals, in turn, to be partially or fully halogenated and/or to have attached to them one to three C₁-C₄-alkyl groups;
excluding hydroximic acid derivatives of the formula I where
a)
X is NO and
Y is O or NH₃ or
b)
X is CHO and
Y is O;
R¹, R³ are methyl; and
R² is ethyl, 2,2,2-trifluoroethyl, 2-chloroethyl, n-propyl, isopropyl, tert-pentyl or n-hexyl.

2. A compound of the formula I as claimed in claim 1 where R² has the following meaning:
C₂-C₆-alkyl, C₃-C₆-alkenyl or C₃-C₆-alkynyl, it being possible for these groups to be partially or fully halogenated and/or to have attached to them one to three of the following radicals:
- C₃-C₆-cycloalkyl, phenyl or oxiranyl, it being possible for the cyclic radicals, in turn, to be partially or fully halogenated and/or to have attached to them one to three of the following of the following groups: cyano, C₁-C₄-alkyl, C₁-C₄-haloalkyl, C₁-C₆-alkoxy, C₁-C₆-haloalkoxy, C₁-C₆-alkoxycarbonyl, C₁-C₆-alkylthio, C₁-C₆-haloalkylthio and phenyl;
C₃-C₆-cycloalkyl, it being possible for these groups to be partially or fully halogenated and/or to have attached to them one to three of the following radicals:
- cyano, C₁-C₄-alkyl, C₁-C₄-haloalkyl, C₁-C₆-alkoxy, C₁-C₆-haloalkoxy, C₁-C₆-alkylthio, C₁-C₆-haloalkylthio.

3. A process for the preparation of the compounds of the formula I as claimed in claim 1, which comprises reacting, in a manner known per se, a benzyl derivative of the formula II 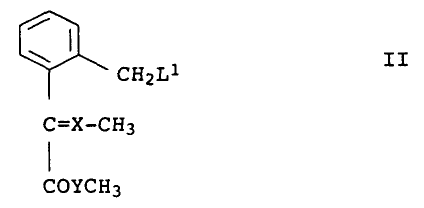 where L¹ is a nucleophilically exchangeable leaving group with a hydroxyimine of the formula III

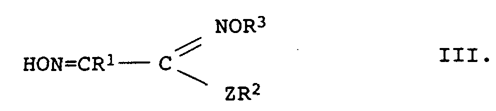

4. A process for the preparation of the compounds of the formula I as claimed in claim 1 which comprises reacting, in a manner known per se, a benzyl derivative of the formula II as claimed in claim 3 with a dihydroxyimine of the formula IV 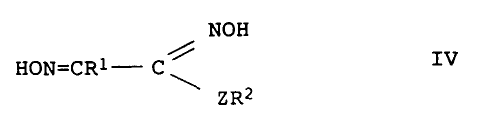 to give a compound of the formula V 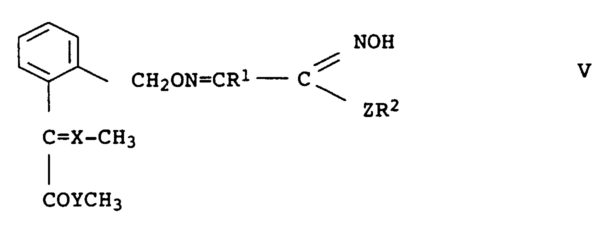 and subsequently reacting V with a compound of the formula VIa
R³-L² VIa
where L² is a nucleophilically exchangeable leaving group, to give I.

5. A composition suitable for controlling animal or fungal pests, comprising a solid or liquid carrier and a compound of the general formula I as claimed in claim 1.

6. A method of controlling fungal pests, which comprises treating the fungi or the materials, plants, soils or seeds to be protected against fungal infection with an effective amount of a compound of the general formula I as claimed in claim 1.

7. A method of controlling animal pests, which comprises treating the pests or the materials, plants, soils or seeds to be protected against them with an effective amount of a compound of the general formula I as claimed in claim 1.

8. A compound of the formula III 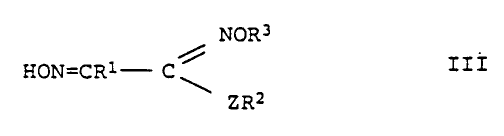 where the substituents Z and R¹ to R³ have the meanings given in claim 1.

9. The use of the compounds III as claimed in claim 3 as intermediates.

10. A compound of the formula Xa 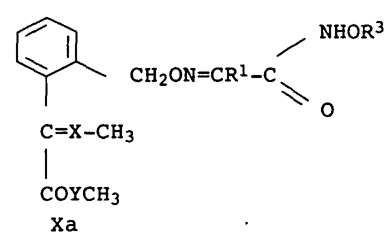 where the substituents X, Y, R¹ and R³ have the meanings given in claim 1.

11. The use of the compounds Xa as claimed in claim 10 as intermediates.

## Revendications

1. Dérivés d'acides hydroximiques de formule I 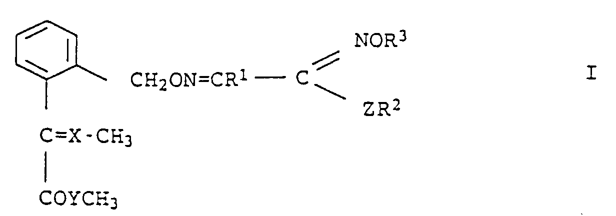 dans laquelle les symboles ont les significations suivantes :
X : CH, CHO ou NO ;
Y : O ou NH ;
Z : O ou S ;
R₁ : l'hydrogène, un groupe alkyle en C1-C6 ou halogénoalkyle en C1-C4 ;
R² : un groupe alkyle en C2-C6, alcényle en C3-C6 ou alcynyle en C3-C6, chacun de ces groupes pouvant être partiellement ou totalement halogéné et/ou pouvant porter un à trois des substituants suivants :
- cyano, nitro, amino, hydroxy,
- alcoxy en C1-C6, halogénoalcoxy en C1-C6, alkylthio en C1-C6, halogénoalkylthio en C1-C6, alkylsulfonyle en C1-C6, alkylsulfoxyde en C1-C6, alkylamino en C1-C6, di-(alkyle en C1-C6)amino,
- cycloalkyle en C3-C6, aryle, hétéroaryle ou oxiranyle, les radicaux cycliques pouvant eux-mêmes être partiellement halogénés et/ou pouvant porter un à trois des substituants suivants : cyano, nitro, amino, aminocarbonyle, aminothiocarbonyle, alkyle en C1-C4, halogénoalkyle en C1-C4, (alkyle en C1-C4)carbonyle, alcoxy en C1-C6, halogénoalcoxy en C1-C6, (alcoxy en C1-C6)carbonyle, alkylthio en C1-C6, halogénoalkylthio en C1-C6, alkylsulfonyle en C1-C6, alkylsulfoxyde en C1-C6, alkylamino en C1-C6, di-(alkyle en C1-C6)amino, (alkyle en C1-C6)aminocarbonyle, di-(alkyle en C1-C6)aminocarbonyle, alcényloxy en C2-C6 et phényle ;
un groupe cycloalkyle en C3-C6, lequel peut être partiellement ou totalement halogéné et/ou peut être porter un à trois des substituants suivants :
- cyano, nitro amino, hydroxy,
- alkyle en C1-C4, halogénoalkyle en C1-C4, alcoxy en C1-C6, halogénoalcoxy en C1-C6, alkylthio en C1-C6, halogénoalkylthio en C1-C6, alkylsulfonyle en C1-C6, alkylsulfoxyde en C1-C6, alkylamino en C1-C6, di-(alkyle en C1-C6)amino;
R³ : un groupe alkyle en C1-C6, alcényle en C2-C6 ou alcynyle en C2-C6, chacun de ces groupes pouvant être partiellement ou totalement halogéné et/ou pouvant porter un à trois des substituants suivants :
- cyano, alcoxy en C1-C3,
- oxiranyle ou cycloalkyle en C3-C6, les groupes cycliques pouvant eux-mêmes être partiellement ou totalement halogénés et/ou pouvant porter un à trois groupes alkyle en C1-C4;
à l'exception des dérivés d'acides hydroximiques de formule I dans laquelle
a) X représente NO et
Y représente O ou NH, ou bien
b) X représente CHO et
Y représente O;
R¹ et R³ sont des groupes méthyle ; et
R² représente un groupe éthyle, 2,2,2-trifluoréthyle, 2-chloréthyle, n-propyle, isopentyle, tert-pentyle ou n-hexyle.

2. Composés de formule I selon la revendication 1, dans laquelle R² a les significations suivantes :
alkyle en C2-C6, alcényle en C3-C6 ou alcynyle en C3-C6, chacun de ces groupes pouvant être partiellement ou totalement halogéné et/ou pouvant porter un à trois des substituants suivants :
- cycloalkyle en C3-C6, phényle ou oxiranyle, les groupes cycliques pouvant eux-mêmes être partiellement ou totalement halogénés et/ou pouvant porter un à trois des substituants suivants : cyano, alkyle en C1-C4, halogénoalkyle en C1-C4, alcoxy en C1-C6, halogénoalcoxy en C1-C6, (alcoxy en C1-C6)carbonyle, alkylthio en C1-C6, halogénoalkylthio en C1-C6 et phényle ;
cycloalkyle en C3-C6, ce groupe pouvant être partiellement ou totalement halogéné et/ou pouvant porter un à trois des substituants suivants :
- cyano, alkyle en C1-C4, halogénoalkyle en C1-C4, alcoxy en C1-C6, halogénoalcoxy en C1-C6, alkylthio en C1-C6, halogénoalkylthio en C1-C6.

3. Procédé de préparation des composés de formule I de la revendication 1, caractérisé par le fait que l'on fait réagir, de manière connue en soi, un dérivé benzylique de formule II 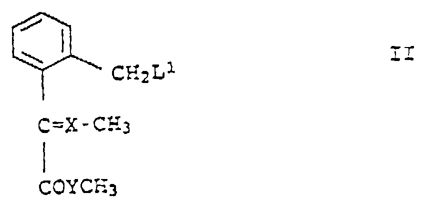 dans laquelle L¹ représente un groupe éliminable par échange nucléophile, avec une hydroxyimine de formule III

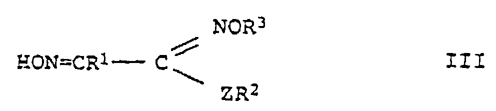

4. Procédé de préparation des composés de formule I de la revendication 1, caractérisé par le fait que l'on fait réagir un dérivé benzylique de formule II de la revendication 4, de manière connue en soi, avec une dihydroxyimine de formule IV 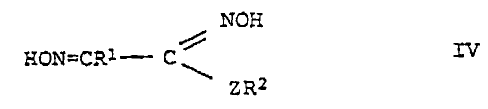 la réaction donnant un composé de formule V 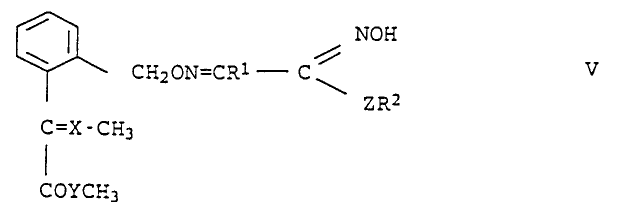 qu'on fait ensuite réagir avec un composé de formule VIa
R³-L² VIa
dans laquelle L² représente un groupe éliminable par échange nucléophile, ce qui donne les composés de formule I.

5. Produit approprié à l'utilisation pour la lutte contre les parasites animaux ou les mycètes nuisibles, contenant un véhicule solide ou liquide et un composé de formule générale I selon la revendication 1.

6. Procédé pour combattre les mycètes nuisibles, caractérisé par le fait que l'on traite les mycètes ou les matières, végétaux, sols ou semences menacés d'une attaque par les mycètes par une quantité efficace d'un composé de formule générale I de la revendication 1.

7. Procédé pour combattre les parasites animaux, caractérisé par le fait que l'on traite les parasites ou les matières, végétaux, sols ou semences menacés d'une attaque par les parasites par une quantité efficace d'un composé de formule générale I de la revendication 1.

8. Composés de formule III 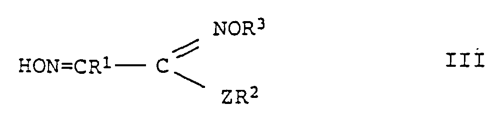 dans laquelle les symboles Z et R¹ à R³ ont les significations indiquées dans la revendication 1.

9. Utilisation des composés III de la revendication 3 en tant que produits intermédiaires.

10. Composés de formule Xa 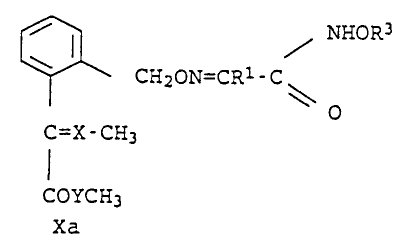 dans laquelle les symboles X, Y, R¹ et R³ ont les significations indiquées dans la revendication 1.

11. Utilisation des composés Xa de la revendication X en tant que produits intermédiaires.
